(19) Europäisches Patentamt
European Patent Office
Office européen des brevets

(11) **EP 3 177 336 B1**

(12) **EUROPÄISCHE PATENTSCHRIFT**

(45) Veröffentlichungstag und Bekanntmachung des Hinweises auf die Patenterteilung:
**11.03.2020 Patentblatt 2020/11**

(21) Anmeldenummer: **15738931.3**

(22) Anmeldetag: **20.07.2015**

(51) Int Cl.:
**A61M 1/16** (2006.01)

(86) Internationale Anmeldenummer:
**PCT/EP2015/066582**

(87) Internationale Veröffentlichungsnummer:
**WO 2016/020180 (11.02.2016 Gazette 2016/06)**

(54) **VORRICHTUNG UND VERFAHREN ZUR ERMITTLUNG EINES OPTIMALEN DIALYSATFLUSSES FÜR EINE EXTRAKORPORALE BLUTBEHANDLUNG MIT EINER EXTRAKORPORALEN BLUTBEHANDLUNGSVORRICHTUNG**

DEVICE AND METHOD FOR DETERMINING AN OPTIMUM DIALYSATE FLOW FOR AN EXTRACORPOREAL BLOOD TREATMENT WITH AN EXTRACORPOREAL BLOOD TREATMENT DEVICE

DISPOSITIF ET PROCÉDÉ SERVANT À DÉTERMINER UN FLUX OPTIMAL DE DIALYSAT EN VUE D'UN TRAITEMENT DU SANG EXTRACORPOREL À L'AIDE D'UN DISPOSITIF DE TRAITEMENT DU SANG EXTRACORPOREL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.08.2014 DE 102014011699**

(43) Veröffentlichungstag der Anmeldung:
**14.06.2017 Patentblatt 2017/24**

(73) Patentinhaber: **Fresenius Medical Care Deutschland GmbH**
**61352 Bad Homburg (DE)**

(72) Erfinder:
• **KOPPERSCHMIDT, Pascal**
**97456 Dittelbrunn (DE)**
• **MAIERHOFER, Andreas**
**97422 Schweinfurt (DE)**
• **GAGEL, Alfred**
**96123 Litzendorf (DE)**
• **WÜPPER, Andreas**
**64572 Büttelborn (DE)**
• **MOISSL, Ulrich**
**61184 Karben (DE)**

(74) Vertreter: **Oppermann, Frank**
**OANDO Oppermann & Oppermann LLP**
**Wilhelminenstrasse 1a**
**65193 Wiesbaden (DE)**

(56) Entgegenhaltungen:
**EP-A1- 2 514 449      WO-A1-2007/140993**
**US-A- 5 100 554      US-A1- 2010 042 035**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Blutbehandlungsvorrichtung mit einer Vorrichtung zur Ermittlung eines optimalen Dialysatflusses sowie ein Verfahren zur Ermittlung eines optimalen Dialysatflusses für eine extrakorporale Blutbehandlung mit einer extrakorporalen Blutbehandlungsvorrichtung. Die Offenbarung bezieht sich auf alle Verfahren der Blutreinigungstherapie, bei denen Blut durch die Blutkammer und Dialysat durch die Dialysatkammer eines Dialysators oder Filters strömt, insbesondere die Hämodialyse oder Hämodiafiltration.

Es sind verschiedene physikalische und/oder chemische Größen bekannt, mit denen die Leistungsfähigkeit eines Dialysators und/oder die Effektivität einer Dialysebehandlung angegeben werden können. Eine bekannte Größe zur Angabe der Effektivität einer Dialysebehandlung stellt die Clearance K dar. Die Clearance K einer Substanz ist der Teilstrom des Gesamtstroms durch den Dialysator, der von der betreffenden Substanz vollständig befreit wird. Für die Effektivität einer Dialysebehandlung ist die sogenannte Dialysedosis KT/V von entscheidender Bedeutung, die als der Quotient aus dem Produkt von Clearance K für Harnstoff und effektiver Behandlungszeit T der Dialysebehandlung und dem Verteilungsvolumen V des Patienten für Harnstoff definiert ist.

Verfahren und Vorrichtungen zur Messung der Clearance während einer extrakorporalen Blutbehandlung sind aus der DE 39 38 662 A1 (US 5,100,554) und DE 197 47 360 A1 (US 6 156 002) bekannt. Die Bestimmung der Clearance beruht auf der Messung des Elektrolyttransfers bei zwei unterschiedlichen Dialysat-Ionenkonzentrationen. Aus den Druckschriften ist bekannt, dass die Clearance vom Dialysatfluss abhängig ist. Die Clearance ist auch vom Blutfluss abhängig, wobei korrekterweise nur der effektive Blutwasserfluss (Plasmawasser und intrazelluläres Wasser) entscheidend ist. Ein niedriger Blutwasserfluss begrenzt die Clearance ungeachtet eines deutlich höheren Dialysatflusses. Liegt der Dialysatfluss unter dem Blutwasserfluss, begrenzt der Dialysatfluss die Clearance. Zwischen Blutwasserfluss und Blutfluss wird der Einfachheit halber nachfolgend nicht mehr unterschieden.

Die bekannten Dialysegeräte erlauben die manuelle Einstellung unterschiedlicher Dialysatraten, beispielsweise 300, 500 und 800 ml/min. Zur Erzielung einer hohen Clearance sind grundsätzlich höhere Dialysatflüsse bei höheren Blutflüssen erforderlich.

Bei der Einstellung eines bestimmten Dialysatflusses ist zu berücksichtigen, dass mit einem hohen Dialysatfluss zwar gegebenenfalls eine hohe Clearance erzielt werden kann, sich aber die Kosten für die Bereitstellung frischer und die Entsorgung verbrauchter Dialysierflüssigkeit erhöhen. Daher wird in der Praxis eine verhältnismäßig hohe Clearance bei relativ niedrigem Verbrauch an Dialysat angestrebt.

Die US 5,092,836 schlägt vor, den Dialysatfluss in Abhängigkeit vom Blutfluss nach vorgegebenen Kriterien zu steuern. Es wird insbesondere vorgeschlagen, einen Dialysatfluss einzustellen, der sich durch Multiplikation des Blutflusses mit einem konstanten Faktor ergibt. Neben einem linearen Zusammenhang zwischen Blut- und Dialysatfluss wird ein numerisches Datenfeld vorgeschlagen, das zu jedem Blutfluss eines bestimmten Dialysators denjenigen Dialysatfluss angibt, bei dem ein bestimmter Prozentsatz der maximalen Clearance, die unter der Annahme eines unendlich hohen Dialysatflusses vorliegen müsste, erreicht wird.

Aus der DE 10 2006 045 437 A1 (US 2010/042035A1) ist eine Vorrichtung zur Ermittlung eines optimalen Dialysatflusses auf der Grundlage eines die Abhängigkeit der Clearance vom Dialysatfluss beschreibenden Zusammenhangs bekannt. Die Ermittlung des optimalen Dialysatflusses beruht darauf, bei einem vorgegeben Blutfluss denjenigen Dialysatfluss zu ermitteln, bei dessen Erhöhung um einen bestimmten Wert die Erhöhung der Clearance einen bestimmten Wert nicht unterschreitet. Der optimale Dialysatfluss ist aber auch von dem Dialysator abhängig, der für die Dialysebehandlung eingesetzt wird. Daher sieht die DE 10 2006 045 437 A1 vor, eine für den Dialysator charakteristischen Größe, insbesondere den Massentransferkoeffizienten, zu berücksichtigen. Der Massentransferkoeffizient stellt eine vom Hersteller der Dialysatoren angegebene Kenngröße für den Dialysator dar, die zur Ermittlung des optimalen Dialysatflusses nach dem bekannten Verfahren eingegeben werden soll. Die DE 10 2006 045 437 A1 sieht vor, für verschiedene Typen von Dialysatoren unterschiedliche Massentransferkoeffizienten zu berücksichtigen.

Die WO 2007/140993 A1 beschreibt eine Vorrichtung zur Steuerung einer extrakorporalen Blutbehandlungsvorrichtung, bei der für eine vorgegebene Dialysierflüssizkeitsrate allein auf der Grundlage einer vorgegebenen Abhängigkeit der Clearance von der Blutflussrate und Dialysierflüssigkeitsrate die Blutflussrate ermittelt wird, bei der mit der vorgegebenen Dialysierflüssigkeitsrate eine vorgegebene Clearance beibehalten wird und/oder bei der für eine vorgegebene Blutflussrate allein auf der Grundlage einer vorgegebenen Abhängigkeit der Clearance von der Blutflussrate und Dialysierflüssigkeitsrate die Dialysierflüssigkeitsrate ermittelt wird, bei der mit der vorgegebenen Blutflussrate eine vorgegebene Clearance beibehalten wird. Der die Abhängigkeit der Clearance von der Dialysierflüssigkeitsrate beschreibende Zusammenhang ist eine den Massentransferkoeffizienten des Dialysators berücksichtigende Funktion.

Der Erfindung liegt die Aufgabe zu Grunde, eine Blutbehandlungsvorrichtung mit einer Vorrichtung zur Ermittlung eines optimalen Dialysatflusses und ein Verfahren zur Ermittlung eines optimalen Dialysatflusses für eine extrakorporale Blutbehandlung mit einer extrakorporalen Blutbehandlungsvorrichtung unter Berücksichtigung des für die Blutbehandlung verwendeten Dialysators anzugeben, wobei einerseits der Forderung nach einer hohen Effektivität der Dialysebehandlung und andererseits nach einem geringen Verbrauch von Dialysat Rechnung getragen wird. Eine weitere Aufgabe der Erfindung liegt darin, eine Blutbehandlungsvorrichtung bereitzustellen, mit der eine Dialysebehandlung mit verhältnismäßig hoher Effektivität bei einem relativ geringen Dialysatfluss durchgeführt werden kann. Eine Aufgabe der

Erfindung ist auch, ein Verfahren zur Ermittlung eines optimalen Dialysatflusses anzugeben, um eine Dialysebehandlung mit einer relativ hohen Effektivität bei einem angemessenen Verbrauch von Dialysat durchführen zu können.

Die Lösung dieser Aufgaben erfolgt erfindungsgemäß mit den Merkmalen der unabhängigen Patentansprüche. Die abhängigen Ansprüche betreffen vorteilhafte Ausführungsformen der Erfindung.

Die erfindungsgemäße Vorrichtung zur Ermittlung eines optimalen Dialysatflusses verfügt über eine Rechen- und/oder Auswerteinheit, die derart konfiguriert ist, dass für den Dialysator der Blutbehandlungsvorrichtung die optimale Dialysatrate aus einem die Abhängigkeit der Clearance von dem Dialysatfluss beschreibenden Zusammenhang bestimmt wird.

In diesem Zusammenhang wird unter Rechen- und/oder Auswerteinheit jede Einheit verstanden, die Signale oder Daten empfängt und/oder auswertet und/oder Signale oder Daten erzeugt bzw. liefert. Die Rechen- und/oder Auswerteinheit kann eine zentrale Einheit sein oder mehrere separate Komponenten umfassen. Sie kann beispielsweise eine Datenverarbeitungseinheit (Mikroprozessor) mit einer Speichereinheit sein, auf der ein Datenverarbeitungsprogramm (Software) läuft.

Die Erfindung hat zwei Aspekte, die unabhängig voneinander von erfinderischer Bedeutung sind. Beide Aspekte beruhen aber auf der Messung der Clearance vor oder während der Blutbehandlung zur Bestimmung des optimalen Dialysatflusses.

Die erfindungsgemäße Vorrichtung weist eine Messeinrichtung zum Messen von mindestens einer für die Clearance charakteristischen Größe auf, wobei die Rechen- und/oder Auswerteinheit derart konfiguriert ist, dass auf der Grundlage der mindestens einen für die Clearance charakteristischen Größe die Clearance ermittelt werden kann.

Die Rechen- und/oder Auswerteinheit der erfindungsgemäßen Vorrichtung ist derart konfiguriert, dass der optimale Dialysatfluss aus dem die Abhängigkeit der Clearance von dem Dialysatfluss beschreibenden Zusammenhangs auf der Grundlage der gemessenen Clearance ermittelt wird. Wenn die Clearance bekannt ist, die sich bei einem bestimmten Dialysatfluss einstellt, kann die für den jeweils verwendeten Dialysator charakteristische Kenngröße, insbesondere der Massentransferkoeffizient des Dialysators, bestimmt werden, der einen Einfluss auf die Effizienz der Blutbehandlung ausübt. Folglich braucht nicht der vom Hersteller vorgegebene Massentransferkoeffizient angenommen zu werden. Die Kenntnis einer derartigen Kenngröße für den verwendeten Dialysator ist also für die erfindungsgemäße Vorrichtung und das erfindungsgemäße Verfahren nicht notwendig.

Es hat sich gezeigt, dass die Messung der Clearance anstelle der Verwendung einer vom Hersteller vorgegebenen Kenngröße für den Dialysator für die Ermittlung des optimalen Dialysatflusses auf der Grundlage des Zusammenhangs zwischen Dialysatfluss und Clearance den Vorteil hat, dass auch Veränderungen der Eigenschaften des Dialysators während der Blutbehandlung aufgrund des Zusetzens der Membran (Clotting) erfasst werden. Darüber hinaus lässt sich der optimale Dialysatfluss selbst dann genau bestimmen, wenn der vom Hersteller vorgegebene Massentransferkoeffizient beispielsweise wegen Fertigungstoleranzen den Dialysator nur unzureichend kennzeichnen sollte.

Die Erfindung beruht darauf, dass für verschiedene Dialysatoren mit unterschiedlichen Kenngrößen oder einen Dialysator, dessen Kenngröße sich verändert, die Abhängigkeit der Clearance vom Dialysatfluss immer von einer charakteristischen Kurve beschrieben wird. Für den Fall verschiedener Dialysatoren oder den Fall sich verändernder Dialysatoreigenschaften ergibt sich also eine Kurvenschar.

Der erste Aspekt der Erfindung liegt darin, mit der gemessenen Clearance die betreffende Kurve aus der Kurvenschar auszuwählen, die für die Bestimmung eines optimalen Arbeitspunktes herangezogen wird.

Eine bevorzugte Ausführungsform der Erfindung sieht als Kenngröße des Dialysators dessen Massentranferkoeffizienten vor. Die Rechen- und/oder Auswerteinheit ist vorzugsweise derart konfiguriert ist, dass der Massentransferkoeffizient $K_O A$ des Dialysators für den Fall der Hämodialyse (HD) nach der folgenden Gleichung berechnet wird:

$$K_O A = \frac{Q_b \cdot Q_d}{Q_b - Q_d} \ln\left(\frac{Q_d(Q_b - K)}{Q_b(Q_d - K)}\right) \qquad \text{Gleichung (1)}$$

,wobei $Q_d$ der Dialysatsfluss, $Q_b$ der Blut(wasser)fluss und K die gemessene Clearance ist.

Nach der Berechnung des Massentransferkoeffizienten $K_O A$ auf der Grundlage der Clearancemessung vor oder während der Blutbehandlung wird der die Abhängigkeit der Clearance vom Dialysatfluss beschreibende Zusammenhang, d. h. die charakteristische Kurve zur Festlegung des optimalen Arbeitspunktes bestimmt. Die Rechen- und/oder Auswerteinheit ist vorzugsweise derart konfiguriert, dass der die Abhängigkeit der Clearance von der Dialysatsrate beschreibende Zusammenhang für den Fall der Hämodialyse (HD) auf der Grundlage der folgenden Gleichung bestimmt wird:

$$K = Q_b \frac{e^{K_0 A\left(\frac{1}{Q_b} - \frac{1}{Q_d}\right)} - 1}{e^{K_0 A\left(\frac{1}{Q_b} - \frac{1}{Q_d}\right)} - \frac{Q_b}{Q_d}}$$

Gleichung (2)

,wobei $Q_d$ der Dialysatfluss, $Q_b$ der Blut(wasser)fluss und $K_0A$ der Massentransferkoeffizient des Dialysators ist.

Eine bevorzugte Ausführungsform sieht für die Bestimmung des optimalen Arbeitspunktes auf der charakteristischen Kurve vor, dass die Rechen- und/oder Auswerteinheit derart konfiguriert ist, dass auf der Grundlage des die Abhängigkeit der Clearance von dem Dialysatfluss beschreibenden Zusammenhangs bei einer vorgegebenen Blutflussrate $Q_b$ derjenige Dialysatfluss $Q_d$ ermittelt wird, bei dessen Erhöhung um einen bestimmten Wert die Erhöhung der Clearance einen bestimmten Wert nicht unterschreitet.

Die erfindungsgemäße Vorrichtung und das erfindungsgemäße Verfahren gehen dabei davon aus, dass ab einem optimalen Wert für den Dialysatfluss bei einer vorgegebenen Blutflussrate zwar mit einer weiteren Erhöhung der Dialysatsrate noch eine Erhöhung der Effektivität der Dialysebehandlung erzielt werden kann, das zusätzliche Dialysat, das für eine derartige Behandlung mit höherer Effektivität erforderlich ist, aber nicht in einem wirtschaftlichen Verhältnis zu der damit verbundenen Erhöhung der Effektivität steht. Es wird also als Zielkriterium der Arbeitspunkt gesucht, bei dem der Verbrauch an zusätzlichem Dialysat, der für die Erhöhung der Clearance um einen bestimmten Wert erforderlich wäre, einen bestimmten Wert nicht überschreitet. Dieses Verfahren ist in der DE 10 2006 045 437 A1 beschrieben, auf die ausdrücklich Bezug genommen wird.

Die erfindungsgemäße Vorrichtung kann eine separate Einrichtung sein oder Bestandteil der Blutbehandlungsvorrichtung sein. Folglich kann auch die Rechen- und/oder Auswerteinheit eine separate Einheit oder Teil der zentralen Steuer- oder Rechen- oder Auswerteinheit der Blutbehandlungsvorrichtung sein.

Ein anderer Aspekt der Erfindung liegt darin, zur Bestimmung des optimalen Dialysatflusses auf der Grundlage zumindest einer Clearance-Messung den optimalen Arbeitspunkt auf der charakteristischen Kurve in einem vorzugsweise iterativen Verfahren festzulegen.

Die Rechen- und/oder Auswerteinheit ist bei einer bevorzugten Ausführungsform derart konfiguriert ist, dass die Differenz von dem Wert der bei einem vorgegebenen Dialysatfluss gemessenen Clearance und einem vorgegebenen Wert für die Clearance berechnet wird, wobei ein Steuersignal zur Verringerung des Dialysatflusses um einen vorgegebenen Betrag erzeugt wird, wenn die Differenz zwischen der gemessenen Clearance und der vorgegebenen Clearance positiv ist, und ein Steuersignal zur Erhöhung des Dialysatflusses um einen vorgegebenen Betrag erzeugt wird, wenn die Differenz zwischen der gemessenen Clearance und der vorgegebenen Clearance negativ ist.

Die Clearance kann vom Arzt im Hinblick auf das Therapieziel unter Berücksichtigung der möglichen Behandlungsparameter frei vorgegeben werden. Die Clearance kann einem Wert entsprechen, der auf jeden Fall mit der Blutbehandlung erzielt werden sollte. Sie kann auch einem Wert entsprechen, der nicht überschritten werden soll, insbesondere bei Patienten, die sich zum ersten Mal einer Dialysebehandlung unterziehen. Die Steuersignale dienen dazu, einen Eingriff in die Maschinensteuerung der Blutbehandlungsvorrichtung vorzunehmen, um die jeweiligen Flussraten einzustellen.

Der Betrag, um den der Dialysatfluss verringert oder erhöht wird, ist vorzugsweise ein von dem Betrag der Differenz von gemessener bzw. berechneter und vorgegebener Clearance abhängiger Betrag. Je größer die Abweichung ist desto größer sollte die Veränderung des Dialysatflusses sein.

Bei einer besonders bevorzugten Ausführungsform erfolgt die Clearancemessung und die Berechnung der Differenz von gemessener und vorgegebener Clearance sowie die Erzeugung eines Steuersignals zur Erhöhung oder Verringerung des Dialysatflusses vorzugsweise in mehreren aufeinanderfolgenden Schritten, so dass der optimale Arbeitspunkt in einem iterativen Prozess ermittelt wird. Zur Vermeidung von ständigen Flussänderungen kann vorgesehen sein, dass der Dialysatfluss nur so lange erhöht bzw. verringert wird, bis der Betrag der Differenz zwischen der aktuell gemessenen Clearance und der vorgegebenen Clearance einen vorgegebenen Grenzwert erreicht oder unterschreitet.

Die erfindungsgemäße Vorrichtung sowie das erfindungsgemäße Verfahren können dazu Verwendung finden, dem behandelnden Arzt bei der Dialysebehandlung einen Vorschlag für die Einstellung eines optimalen Dialysatflusses zu geben. Weiterhin bevorzugt ist, dass die erfindungsgemäße Vorrichtung nicht nur einen Vorschlag für die Einstellung eines optimalen Dialysatflusses gibt, sondern auch derart konfiguriert ist, dass eine automatische Einstellung des Dialysatflusses vorgesehen wird.

Eine alternative bevorzugte Ausführungsform sieht eine sukzessive Erhöhung oder Verringerung bzw. die Beibehaltung des Dialysatflusses in Abhängigkeit von der Erfüllung eines vorgegebenen Kriteriums vor. Der Dialysatfluss wird bei der bevorzugten Ausführungsform beispielsweise solange sukzessive erhöht, wie ein bestimmtes Kriterium erfüllt ist, beispielsweise wird überprüft, ob die Erhöhung von dem vorausgehenden Wert auf den nachfolgenden Wert zu einer Erhöhung der Cleareance geführt hat, die in einem bestimmten Verhältnis zu der Erhöhung des Dialysatflusses steht.

Im Folgenden werden verschiedene Ausführungsbeispiele der Erfindung unter Bezugnahme auf die Zeichnungen näher erläutert.

Es zeigen:

Figur 1  Die wesentlichen Komponenten einer erfindungsgemäßen Vorrichtung zur extrakorporalen Blutbehandlung mit einer erfindungsgemäßen Vorrichtung zur Vorgabe eines optimalen Dialysatflusses in stark vereinfachter schematischer Darstellung,

Figur 2  die Clearance K (ml/min) als Funktion des Dialysatflusses $Q_d$ (ml/min) für verschiedene Blutflussraten $Q_b$, und

Figur 3  ein Flussdiagramm mit den Verfahrensschritten zur Ermittlung eines optimalen Dialysatflusses $Qd_{opt}$ in einem iterativen Verfahren.

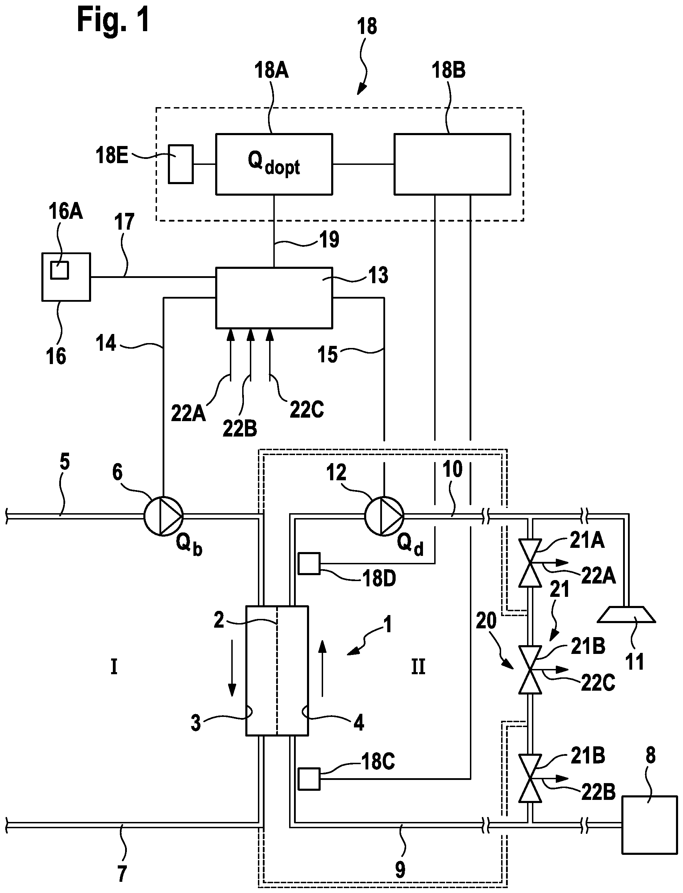

Figur 1 zeigt ein Ausführungsbeispiel einer erfindungsgemäßen Blutbehandlungsvorrichtung, die über eine erfindungsgemäße Vorrichtung 18 zur Ermittlung eines optimalen Dialysatflusses $Qd_{opt}$ verfügt. In Fig. 1 sind der besseren Übersichtlichkeit halber nur die wesentlichen Komponenten der Blutbehandlungsvorrichtung dargestellt, da dem Fachmann die einzelnen Komponenten einer Blutbehandlungsvorrichtung zur Hämodialyse oder Hämodiafiltration allgemein bekannt sind.

Die erfindungsgemäße Dialysevorrichtung verfügt über einen Dialysator 1, der durch eine semipermeable Membran 2 in eine Blutkammer 3 und eine Dialysatkammer 4 unterteilt ist. Von einem Patienten führt eine arterielle Blutleitung 5, in die eine Blutpumpe 6 geschaltet ist, zu einem Einlass der Blutkammer 3, während von einem Auslass der Blutkammer 3 des Dialysators 1 eine venöse Blutleitung 7 zu dem Patienten führt. Während der Blutbehandlung sind die arterielle und venöse Blutleitung 5, 7 des extrakorporalen Blutkreislaufs I mit nicht dargestellten Kanülen an den Patienten angeschlossen.

In einer Dialysatquelle 8 wird frisches Dialysat bereitgestellt. Von der Dialysatquelle 8 führt eine Dialysatzuführleitung 9 zu einem Einlass der Dialysatkammer 4, während eine Dialysatabführleitung 10 von einem Auslass der Dialysatkammer 4 des Dialysators 1 zu einem Abfluss 11 führt. In die Dialysatabführleitung 10 ist eine Dialysatpumpe 12 geschaltet.

Das Dialysatsystem II der Dialysevorrichtung umfasst noch weitere Komponenten, von denen in Fig. 1 aber nur eine Bypassleitung 20 dargestellt ist, die eine Ventilanordnung 21 aufweist. Das eine Ende der Bypassleitung 20 ist an die Dialysatzuführleitung 9 stromauf der Dialysatkammer 4 und das andere Ende der Bypassleitung 20 ist an die Dialysatabführleitung 10 stromab der Dialysatkammer angeschlossen. Die Ventilanordnung 21 weist zwei äußere Ventile 21A und 21B und ein mittleres Ventil 21C auf, die in der Bypassleitung angeordnet sind.

Die Dialysevorrichtung verfügt über eine zentrale Steuereinheit 13, die mit der Blutpumpe 6 und der Dialysatpumpe 12 über Steuerleitungen 14, 15 verbunden ist. Die Steuereinheit 13 erzeugt Steuersignale zum Betreiben der Blut- und Dialysatpumpe 6, 12 mit einer vorgegebenen Förderrate, so dass sich in der Blutleitung 5, 7 ein vorgegebener Blutfluss $Q_b$ und in der Dialysatleitung 9, 10 ein vorgegebener Dialysatfluss $Q_d$ einstellen. Die Ventile 21A, 21B, 21C sind elektromagnetisch betätigbare Ventile, die über Steuerleitungen 22A, 22B, 22C mit der Steuereinheit 13 verbunden sind

Zur Eingabe verschiedener Parameter für die Dialyse verfügt die Dialysevorrichtung über eine Eingabeeinheit 16, die beispielsweise eine alphanumerische Tastatur 16A aufweist. Die Eingabeeinheit 16 ist über eine Datenleitung 17 mit der Steuereinheit 13 verbunden, mit der die einzelnen Komponenten der Dialysevorrichtung derart angesteuert werden, dass die Dialysebehandlung mit den vorgegebenen Dialyseparametem durchgeführt wird.

Die Dialysevorrichtung gibt für die Dialysehandlung einen optimalen Dialysatfluss $Q_{dopt}$ vor. Hierzu verfügt die Dialysevorrichtung über eine Vorrichtung 18 zur Ermittlung eines optimalen Dialysatflusses $Q_{dopt}$, deren Aufbau und Funktionsweise nachfolgend im Einzelnen beschrieben wird.

Die Dialysebehandlung wird mit einem bestimmten Dialysator 1 durchgeführt, der eine bestimmte Effektivität hat. Die Effektivität des Dialysators 1 wird durch den Massentransferkoeffizienten $k_0A$ angeben, der für die Ermittlung des optimalen Dialysatflusses $Q_{dopt}$ aber nicht bekannt zu sein braucht.

Für den Fall der Hämodialyse (HD) berechnet sich die Clearance K aus dem Blut(wasser)fluss $Q_b$ und dem Dialysatfluss $Q_d$ und dem Massentransferkoeffizienten $k_0A$ des Dialysators 1 nach folgender Gleichung:

$$K = Q_b \frac{e^{K_0 A\left(\frac{1}{Q_b} - \frac{1}{Q_d}\right)} - 1}{e^{K_0 A\left(\frac{1}{Q_b} - \frac{1}{Q_d}\right)} - \frac{Q_b}{Q_d}} \qquad (2)$$

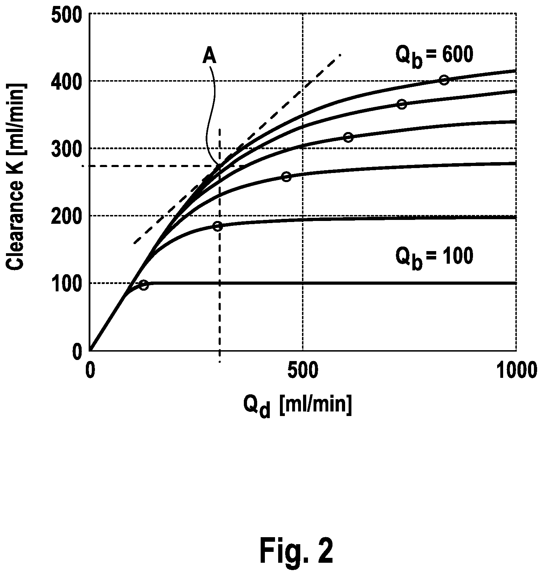

Figur 2 zeigt die Clearance K als Funktion des Dialysatflusses $Q_d$ für verschiedene Blutflüsse $Q_b$. Es zeigt sich, dass bei hohen Dialysatflüssen $Q_d$ eine Sättigung der Clearance K eintritt. Daher führt ab einem gewissen Dialysatfluss $Q_{dopt}$ eine Erhöhung des Dialysatflusses zu keinem nennenswerten Gewinn an Clearance. Ist der Gewinn an Clearance vernachlässigbar, so kann eine Änderung der Dialysatrate unterbleiben bzw. eine Reduktion des Dialysatflusses kann sinnvoll sein. Führt die Änderung der Dialysatrate hingegen zu einer signifikanten Erhöhung der Clearance, so sollte die Dialysatrate erhöht werden. Mittels einer kritischen Größe *crit* kann festgelegt werden, ob der Dialysatfluss reduziert bzw. erhöht werden soll oder unverändert bleiben kann.

$dK(Q_d) / dQ_d < crit_1$       Reduzierung von $Q_d$       Gleichung (3.1)

$crit_1 < dK(Q_d) / dQ_d < crit_2$       $Q_d$ wird nicht verändert       Gleichung (3.2)

$dK(Q_d) / dQ_d > crit_2$       Erhöhung von $Q_d$       Gleichung (3.3)

Die Vorrichtung 18 zur Ermittlung des optimalen Dialysatfluss $Q_{dopt}$ verfügt über eine Rechen- und/oder Auswerteinheit 18A, die über eine Leitung 19 mit der zentralen Steuereinheit 13 der Blutbehandlungsvorrichtung verbunden ist, so dass Rechen- und/oder Auswerteinheit 18A und Steuereinheit 13 Steuersignale oder Daten empfangen bzw. senden können.

Darüber hinaus verfügt die Vorrichtung zur Ermittlung des optimalen Dialysatfluss $Q_{dopt}$ über eine Messeinrichtung 18B zum Messen von einer für die Clearance charakteristischen Größe, insbesondere der Dialysat-Ionenkonzentration am Eingang und Ausgang der Dialysatkammer des Dialysators. Zum Messen der Dialysat-Eingangskonzentration und der Dialysat-Ausgangskonzentration weist die Messeinrichtung 18B einen Leitfähigkeitssensor 18C an der Dialysatzuführleitung 9 stromauf der Dialysatkammer 2 und einen Leitfähigkeitssensor 18D an der Dialysatabführleitung 10 stromab der Dialysatkammer 2 des Dialysators 1 auf, die in Fig. 1 nur andeutungsweise dargestellt sind. Die Messung der Clearance K beruht darauf, dass die Elektrolytkonzentration kurzzeitig angehoben oder abgesenkt wird, wobei vor der Veränderung der Elektrolytkonzentration die Leitfähigkeit des Dialysats stromauf bzw. stromab der Dialysatkammer 2 mit den Sensoren 18C, 18D gemessen wird. Aus den Dialysat-Eingangskonzentrationen $c_{di}(1)$ und $c_{di}(2)$ und Dialysat-Ausgangskonzentration $c_{do}(1)$ und $c_{do}(2)$ kann die Rechen- und/oder Auswerteinheit die Clearance nach der folgenden Gleichung berechnen:

$$K = Q_d \left( \left( (c_{di}(1) - c_{do}(1)) - (c_{di}(2) - c_{do}(2)) \right) \right) / (c_{di}(1) - c_{di}(2)) \qquad \text{Gleichung (4)}$$

Derartige Messeinrichtungen sind beispielsweise aus der DE 39 38 662 A1 (US 5,100,554) und DE 197 47 360 A1 (US 6 156 002) bekannt, auf die ausdrücklich Bezug genommen wird. Für die Erfindung ist aber unerheblich wie die Clearance aus den gemessenen Größen berechnet wird.

Die erfindungsgemäße Vorrichtung 18 gibt einen optimalen Dialysatfluss $Q_{dopt}$ vor, mit dem die Dialysevorrichtung betrieben wird. Dabei wird davon ausgegangen, dass ein bestimmter Blutfluss $Q_b$ eingestellt wird, der auf der Eingabeeinheit 16 eingegeben werden kann. Hierfür ist die Rechen- und/oder Auswerteinheit 18A wie folgt konfiguriert.

Bei dem eingestellten Blutfluss $Q_b$ wird zunächst die Clearance K für einen vorgegebenen Dialysatfluss $Q_d$ gemessen. Die Rechen- und/oder Auswerteinheit 18A berechnet nach Gleichung (4) aus den gemessenen Leitfähigkeitswerten vor und nach der Veränderung der Elektrolytkonzentration die Clearance K. Nachdem die Clearance K bekannt ist, berechnet die Rechen- und Auswerteinheit 18A den Massentransferkoeffizienten $k_0 A$ des Dialysators 1 für den Fall der Hämodialyse (HD) nach der Gleichung (1):

$$K_0 A = \frac{Q_b \cdot Q_d}{Q_b - Q_d} \ln\left( \frac{Q_d(Q_b - K)}{Q_b(Q_d - K)} \right)$$

,wobei $Q_d$ der Dialysatsfluss, $Q_b$ der Blut(wasser)fluss und K die zuvor gemessene Clearance ist.

Da der Massentransferkoeffizient $k_0A$ des Dialysators 1 über die Clearancemessung im Vorfeld der Bestimmung des optimalen Arbeitspunktes bestimmt wird, braucht diese Kenngröße des Dialysators nicht bekannt zu sein. Die Dialysatorkenngröße kann vor oder während der Dialysebehandlung ermittelt werden. Während der Dialysebehandlung kann die Kenngröße in bestimmten Zeitintervallen ermittelt werden, so dass auch Änderungen des Massentransferkoeffizienten Berücksichtigung finden können, die auf das Zusetzen der Membran (Clotting) zurückgeführt werden können.

Der Massentransferkoeffizienten braucht mit einer Clearancemessung nicht während der Blutbehandlung bestimmt zu werden, sondern kann auch im Vorfeld der Blutbehandlung während eines Spülvorgangs bestimmt werden.

Zum Spülen der Blutkammer des Dialysators werden die arterielle Blutleitung 5 an den Abschnitt der Bypassleitung 20 zwischen dem einen äußeren Ventil 21A und dem inneren Ventil 21C und die venöse Blutleitung 7 an den Abschnitt der Bypassleitung 20 zwischen dem anderen äußeren Ventil 21B und dem inneren Ventil 21C angeschlossen und von der Steuereinheit 13 werden in der Bypassleitung 20 die äußeren Ventile 21A und 21B geöffnet und das mittlere Ventil 21C geschlossen. Die für den Spülvorgang an die Bypassleitung 20 angeschlossenen Blutleitungen 5, 7 sind in Fig. 1 in gestrichelten Linien gezeichnet. Während des Spülvorgangs wird eine Spülflüssigkeit, insbesondere eine Dialysierflüssigkeit, über die Dialysatzuführleitung 9 und den Abschnitt der venösen Blutleitung 7 der Blutkammer 3 zugeführt und über den Abschnitt der venösen Blutleitung 5 und die Dialysatabführleitung 10 aus der Blutkammer abgeführt. Wenn der Spülvorgang beendet ist, schließt die Steuereinheit 13 die äußeren Ventile 21A, 21B und öffnet das mittlere Ventil 21C in der Bypassleitung 20, wobei die Blutpumpe 6 betrieben wird, so dass die Spülflüssigkeit durch die Blutkammer 3 rezirkuliert. Gleichzeitig wird die Dialysatpumpe 12 betrieben, so dass Diaysat in die Diaysatkammer 4 und aus der Dialysatkammer 4 strömt.

Die Bestimmung der Clearance erfolgt nunmehr nach den oben beschriebenen bekannten Verfahren, wobei anstelle von Blut Spülflüssigkeit, insbesondere eine Dialysierflüssigkeit, durch die Blutkammer strömt. Hierzu wird die Elektrolykonzentration des in die Dialysatkammer 4 strömenden Dialysats kurzzeitig verändert und die Antwort auf den Konzentratbolus in dem aus der Dialysatkammer 4 strömenden Dialysat gemessen. Die Messungen können auch stromauf und stromab des Dialysators erfolgen, wozu die Leitfähigkeitssensoren 18C und 18D verwendet werden können. Aus der Flussrate für die Spülflüssigkeit, die der Blutflussrate $Q_b$ bei einer Messung während der Blutbehandlung entspricht, und der Dialysatflussrate $Q_d$ sowie den gemessenen Leitfähigkeitswerten berechnet die Rechen- und/oder Auswerteinheit 18A nach Gleichung (1) den Massentransferkoeffizienten. Dieser kann für unterschiedliche Dialysatoren und Schlauchleitungssystem bestimmt werden. Bei der Berechnung des Massentransferkoeffizienten nach Gleichung (1) ist aber zu berücksichtigen, dass wegen des im Vergleich zu der Messung während der Blutbehandlung geringeren Volumens der durch die Blutkammer strömenden Flüssigkeit und wegen der Rezirkulation der Flüssigkeit die Gleichung (1) die tatsächlichen Verhältnisse nicht genau beschreibt. Daher nimmt die Rechen- und/oder Auswerteinheit 18A eine Korrektur des berechneten Wertes mit einem empirisch bestimmten Korrekturfaktor vor, der das Füllvolumen der Blutkammer und des Schlauchleitungssystems berücksichtigt. Dieser Korrekturfaktor kann anhand von Laborversuchen ermittelt und in einem Speicher der Rechen- und/oder Auswerteinheit 18A gespeichert werden.

Nachdem der Massentransferkoeffizient $k_0A$ bekannt ist, ermittelt die Rechen- und/oder Auswerteinheit 18A für den Fall der Hämodialyse (HD) nach Gleichung (2) den Zusammenhang zwischen Clearance K und Dialysatfluss $Q_b$:

$$K = Q_b \frac{e^{K_0A\left(\frac{1}{Q_b}-\frac{1}{Q_d}\right)} - 1}{e^{K_0A\left(\frac{1}{Q_b}-\frac{1}{Q_d}\right)} - \frac{Q_b}{Q_d}}$$

,wobei $Q_d$ der Dialysatfluss, $Q_b$ der Blut(wasser)fluss und $K_0A$ der Massentransferkoeffizient des Dialysators ist.

Die Rechen- und/oder Auswerteinheit 18A kann nach Gleichung (1) die Clearance K für unterschiedliche Dialysatflüsse Qd berechnen, um dann den optimalen Arbeitspunkt für die Dialysevorrichtung zu finden, wie in der DE 10 2006 045 437 A1 beschrieben ist.

Die optimalen Arbeitspunkte für unterschiedliche Blutflussraten sind in Figur 2 durch Kreise gekennzeichnet, wobei für die Arbeitspunkte ein Verhältnis von zusätzlichem Dialysatfluss [ml/min] und zusätzlicher Clearance [ml/min] von 10:1 gewählt wurde. Wenn man ausgehend von dem jeweiligen Arbeitspunkt die Dialysatrate $Q_d$ weiter erhöht, so ist eine Erhöhung der Dialysatsrate nicht mehr mit einer weiteren Erhöhung der Clearance K verbunden, die einen bestimmten Wert überschreitet. Die optimale Dialysatrate $Q_{dopt}$ ist also der Dialysatsfluss, bei dessen Überschreiten die Ableitung der in Fig. 2 dargestellten Funktion, mit der die Abhängigkeit der Clearance K von der Dialysatsrate $Q_d$ beschrieben wird, einen bestimmten kritischen Wert unterschreitet. Ein möglicher, aber nicht optimaler Arbeitspunkt ist in Fig. 2 mit A bezeichnet.

Die Clearancemessung im Vorfeld der Bestimmung des optimalen Arbeitspunktes macht die Ermittlung eines dreidimensionales Kennlinienfeldes überflüssig, mit dem sich in Abhängigkeit von der Blutflussrate $Q_b$ für unterschiedliche Dialysatoren, die sich jeweils durch einen bestimmten Massentransferkoeffizienten K0A auszeichnen, die optimale Dialysatflussrate $Q_{dopt}$ bestimmen lässt.

Zur Anzeige des optimalen Dialysatsflusses $Q_{dopt}$ verfügt die Vorrichtung 18 über eine Anzeigeeinheit 18E, beispielsweise in Form eines Bildschirms oder eines Displays.

Darüber hinaus gibt die Vorrichtung 18 den berechneten Wert für den optimalen Dialysatfluss $Q_{dopt}$ über die Leitung 19 an die Steuereinheit 13 der Blutbehandlungsvorrichtung aus, die wiederum die Drehzahl der Dialysatpumpe 12 derart einstellt, dass Dialysat mit dem optimalen Dialysatfluss $Q_{dopt}$ gefördert wird.

Ein anderer Aspekt der Erfindung sieht ebenfalls die Messung der Clearance zur Ermittlung einer optimalen Dialysierflüssigkeitsrate vor.

Der Arzt kann auf der Eingabeeinheit 16 für die Blutbehandlung eine bestimmte Clearance $K_{min}$ vorgeben, die bei der Blutbehandlung nicht unterschritten werden soll. Er kann aber auch einen Wert für die Clearance $K_{max}$ vorgegeben, der nicht überschritten werden soll. Bei einem vorgegebenen Dialysatfluss $Q_d$ wird die Clearance $K_m$ gemessen.

Nachdem die tatsächliche Clearance $K_m$ bekannt ist, berechnet die Rechen- und/oder Auswerteinheit 18A die Differenz von der gemessenen Clearance $K_m$ und der vorgegebenen beispielsweise minimalen Clearance $K_{min}$. Wenn die Differenz positiv ist, erzeugt die Rechen- und/oder Auswerteinheit 18A ein Steuersignal, so dass die Steuereinheit 13 der Blutbehandlungsvorrichtung den Dialysatfluss $Q_d$ verringert. Wenn die Differenz hingegen negativ ist, erzeugt die Rechen- und/oder Auswerteinheit ein Steuersignal für die Steuereinheit zur Erhöhung des Dialysatflusses $Q_d$. Der Betrag $\Delta Q_d$, um den der Dialysatfluss $Q_d$ verringert bzw. erhöht wird, ist proportional zu dem Betrag der Differenz von der gemessenen Clearance $K_m$ und der minimalen Clearance $K_{min}$. Eine große Differenz führt also zu einer starken Veränderung der Dialysatrate, beispielsweise um 20%. Vorzugsweise ist die Abhängigkeit eine lineare Funktion. Die Differenz $K_m$ - $K_{min}$ kann auch auf $K_{min}$ bezogen werden (beispielsweise $(K_m-K_{min})/K_{min}$ x 100% >20%).

Nach der Veränderung des Dialysatflusses $Q_d$ erfolgt wieder eine Clearancemessung, um feststellen zu können, ob das Therapieziel noch erreicht wird. Die Rechen- und/oder Auswerteinheit 18A berechnet die Differenz von der gemessenen Clearance $K_m$ und der minimalen Clearance $K_{min}$. Wenn die Differenz noch positiv ist, erfolgt wieder eine Verringerung des Dialysatflusses $Q_d$ in einem weiteren Schritt. Ist die Differenz hingegen negativ, wird der Dialysatfluss erhöht.

Die Ermittlung des optimalen Dialysatflusses $Q_{dopt}$ kann in mehreren iterativen Schritten erfolgen, wobei die Dialysatrate in jedem Schritt um einen bestimmten Betrag verändert wird, der proportional zu dem Betrag der Differenz von gemessener Clearance $K_m$ und minimaler Clearance $K_{min}$ ist. Zur Vermeidung ständiger Flussratenänderungen vergleicht die Rechen- und/oder Auswerteinheit 18A die Differenz von gemessener Clearance $K_m$ und minimaler Clearance $K_{min}$ mit einem vorgegebenen Grenzwert, der beispielweise zwischen 2% und 5% der minimalen Clearance $K_{min}$ liegen kann. Wenn der Grenzwert erreicht oder unterschritten wird, unterbricht die Rechen- und/oder Auswerteinheit 18A den iterativen Prozess, wobei der gerade eingestellte Dialysatfluss $Q_d$ als der optimale Dialysatfluss $Q_{dopt}$ angenommen wird.

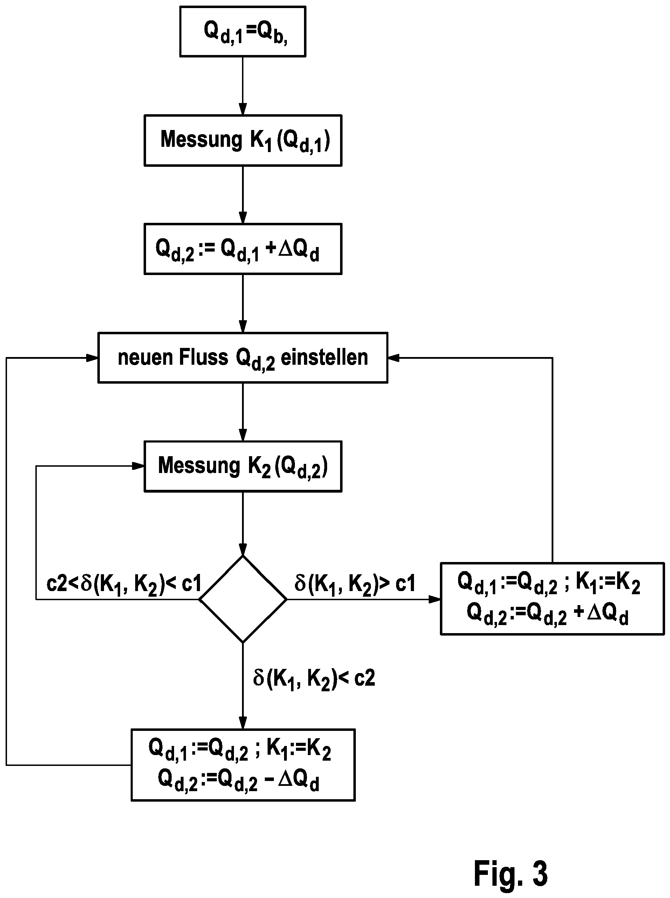

Nachfolgend wird eine weitere Ausführungsform der Erfindung zur Ermittlung des optimalen Dialysatflusses $Q_{dopt}$ mit einem iterativen Verfahren beschrieben, das auf einer Messung der Clearance K beruht. Die einzelnen Verfahrensschritte, die zu Beginn oder im Verlauf der Blutbehandlung durchgeführt werden können, sind in Fig. 3 dargestellt.

Die Steuereinheit 13 erzeugt zunächst ein Steuersignal, das einen dem Blutfluss $Q_b$ entsprechenden Dialysatfluss $Q_{d,1}$ vorgibt. Bei dem vorgegebenen Blutfluss $Q_b$ und Dialysatfluss $Q_{d,1}$ wird die Clearance $K_1$ gemessen, wobei die Bestimmung der Clearance wieder mit dem oben beschriebenen Verfahren erfolgen kann. Dann erhöht die Steuereinheit den Dialysatfluss $Q_{d,1}$ um den Betrag $\Delta Q_d$, beispielsweise um 50 ml/min, auf $Q_{d,2}$. Daraufhin erfolgt eine zweite Messung der Clearance bei dem Blutfluss $Q_b$ mit dem Ergebnis $K_2$. Beide Messungen werden nunmehr von der Rechen- und/oder Auswerteinheit 18A wie folgt ausgewertet.

Die Rechen- und/oder Auswerteinheit 18A berechnet aus den Daten-Tupeln $[K_1, Q_{d,1}]$ und $[K_2, Q_{d,2}]$ die relative Änderung der Clearance von $K_1$ auf $K_2$ infolge der Änderung des Dialysatflusses um $\Delta Q_{d1,2}$ von $Q_{d,1}$ auf $Q_{d,2}$ und berechnet den Gradienten $\Delta K_{12} / \Delta Q_{d12}$ der relativen Änderung der Clearance $\Delta K_{12}$ und des relativen Dialysatflusses $\Delta Q_{d12}$:

$$\Delta K_{12} / \Delta Q_{d12} = (K2 - K1) / (Q_{d,2} - Q_{d,1}) \qquad \text{Gleichung (5)}$$

Die Änderung der Clearance von $K_1$ auf $K_2$ infolge der Änderung des Dialysatflusses um $\Delta Q_{d12}$ von $Q_{d,1}$ auf $Q_{d,2}$ wird von der Rechen- und/oder Auswerteinheit 18A auf die Erfüllung eines Kriteriums für die Erhöhung oder Verringerung oder Beibehaltung des Dialysatflusses $Q_d$ überprüft. Hierfür vergleicht die Rechen- und/oder Auswerteinheit den Gradienten $\Delta K_{12} / \Delta Q_{d12}$ der relativen Änderung der Clearance und des relativen Dialysatflusses mit einem ersten Grenzwert $c_1$ und einem zweiten Grenzwert $c_2$, wobei der erste Grenzwert größer als der zweite Grenzwert ist. Der zweite Grenzwert kann aber gleich dem ersten Grenzwert sein.

Wenn der Gradient $\Delta K_{12} / \Delta Q_{d12}$ größer als der erste Grenzwert $c_1$ ist, erzeugt die Rechen- und Steuereinheit 13 ein Steuersignal zur Erhöhung des Dialysatflusses $Q_d$, so dass der Dialysatfluss $Q_d$ nochmals um $\Delta Q_d$ erhöht wird.

Wenn der Gradient $\Delta K_{12} / \Delta Q_{d12}$ kleiner als der zweite Grenzwert $c_2$ ist, erzeugt die Rechen- und Steuereinheit 13 ein Steuersignal zur Verringerung des Dialysatflusses $Q_d$, so dass der Dialysatfluss $Q_d$ um $\Delta Q_d$ wieder verringert wird.

Wenn der Gradient $\Delta K_{12} / \Delta Q_{d12}$ kleiner als der erste Grenzwert $c_1$ und größer als der zweite Grenzwert $c_2$ ist, wird ein Steuersignal zur Beibehaltung des Dialysatflusses $Q_d$ erzeugt, so dass der optimale Dialysatfluss $Q_{dopt}$ bestimmt und auch eingestellt ist.

Wenn der Gradient $\Delta K_{12} / \Delta Q_{d12}$ größer als der erste Grenzwert $c_1$ oder kleiner als der zweite Grenzwert $c_2$ ist, wird nach der Erhöhung oder Verringerung des Dialysatflusses wieder die Clearance K gemessen, um wieder die Änderung der Clearance von dem zuvor gemessenen Wert auf den aktuellen Wert infolge der Erhöhung oder Verringerung des Dialysatflusses auf die Erfüllung des obigen Kriteriums überprüfen zu können. Dieser Vorgang wird solange fortgesetzt, bis der Gradient $\Delta K_{12} / \Delta Q_{d12}$ kleiner als der erste Grenzwert und größer als der zweite Grenzwert ist und der Dialysatfluss nicht mehr verändert wird.

Die erfindungsgemäße Vorrichtung und das erfindungsgemäße Verfahren stellen auf die Optimierung des Dialysatflusses auf der Grundlage der Bestimmung des diffusiven Anteils an der Dialysatorclearance ab. Die erfindungsgemäße Vorrichtung und das erfindungsgemäße Verfahren können nicht nur für die Ermittlung eines optimalen Dialysatflusses für die Hämodialyse (HD), sondern auch für die Hämodiafiltration (HDF) Verwendung finden. Für den Fall der Hämodiafiltration (HDF) ergeben sich die folgenden Größen:

$K_0A$:   diffusiver Massentransferkoeffizient bzw. Koeffizient des diffusiven Massentransfers, der den diffusiven Anteil der Dialysatorcleacrence berücksichtigt;

$Q_d$:   Dialysatfluss durch den Dialysator, der von dem Gesamtfluss des Dialysats $Q_{d,tot}$ zu unterscheiden ist, der die Summe von dem Dialysatfluss $Q_d$ durch den Dialysator und dem Substituatfluss $Q_s$ ist ($Q_{d,tot} = Q_d + Q_s$);

$Q_{d,opt}$   optimaler Dialysatfluss;

$Q_{bw}$:   Blutwasserfluss an der arteriellen Kanüle. Der Blutfluss am Dialysatoreingang wird durch eine Zufuhr von Substituat stromauf des Dialysators (Prädilution) erhöht, während der Blutfluss am Dialysatorausgang durch eine Ultrafiltration und eine Zufuhr von Substituat stromab des Dialysators (Postdilution) verringert wird. Der Blutwasserfluss $Q_{bw}$ hängt von dem Hämatokrit und dem Proteinanteil im Blut ab, wobei $Q_{bw}$ etwa 0,86 $Q_b$ ist;

$K_{m,tot}$:   gemessene Systemclearance, die die gesamte Reinigungsleistung des Systems einschließlich des konvektiven und diffusiven Anteils der Clearance umfasst, wobei die Clearance vermindernde Patienteneffekte, beispielsweise eine Rezirkulation, Berücksichtigung finden;

$K_{m,diff}$:   diffusiver Anteil der Systemclearance, der von der gemessenen Systemclearance $K_{m,tot}$ abgeleitet wird und auf der Berechnung des diffusiven Massentransferkoeffizienten $K_0A$ beruht.

Als $K_d$ ($Q_d$, $Q_b$, $K_0A$) wird die zur Bestimmung des optimalen Dialysatflusses $Q_{d,opt}$ berechnete Clearance bezeichnet, die von dem Dialysatfluss $Q_d$ und dem Blutfluss $Q_b$ abhängig ist.

Ein Unterschied zwischen der Hämolyse (HD) und der Hämodiafltration (HDF) besteht insbesondere für den Fall der Hämodiafiltration mit Prädilution (HDF-Prädilution), da sich der blutseitige Flüssigkeitsstrom aus dem Blutfluss und dem Substituatfluss zusammensetzt. Für den Summenfluss findet dann der diffusive Austausch im Dialysator statt:

$$K_{m,diff} = \frac{Q_{bw} + \kappa Q_s}{Q_b - Q_f - (1-\kappa)Q_s}\left(\frac{Q_{bw} + \kappa Q_s}{Q_b} K_m - Q_f - Q_s\right),$$

$\kappa = 1$ bei HDF-Prädilution
$\kappa = 0$ bei HD und HDF-Postdilution

Für HDF-Postdilution vereinfacht sich dieser Zusammenhang zu

$$K_{m,diff} = \frac{Q_b}{Q_b - Q_f - Q_s} \left( K_m - Q_f - Q_s \right)$$

Damit ergibt sich anstelle von Gleichung (1) für den Fall der Hämodiafiltration (HDF):

$$k_0 A = \frac{(Q_b + \kappa Q_s) Q_d}{Q_d - Q_b - \kappa Q_s} \ln\left( \frac{\frac{K_{m,diff}}{Q_d} - 1}{\frac{K_{m,diff}}{Q_b + \kappa Q_s} - 1} \right) \qquad \text{Gleichung (1')}$$

wobei sich für den Fall der Hämodialyse oder der HDF-Postdilution mit $\kappa = 0$ ergibt:

$$k_0 A = \frac{Q_b Q_d}{Q_d - Q_b} \ln\left( \frac{\frac{K_{m,diff}}{Q_d} - 1}{\frac{K_{m,diff}}{Q_b} - 1} \right)$$

Der die Abhängigkeit der Clearance $K_d$ von dem Dialysatfluss $Q_d$ beschreibende Zusammenhang ergibt sich für den allgemeinen Fall der Hämodiafiltration (HDF) wie folgt:

$$K_d = (Q_{bw} + \kappa Q_s) \frac{e^\gamma - 1}{e^\gamma - \frac{(Q_{bw} + \kappa Q_s)}{Q_d}}, \gamma = k_0 A \frac{Q_d - (Q_{bw} + \kappa Q_s)}{(Q_{bw} + \kappa Q_s) Q_d} \qquad \text{Gleichung (2')}$$

Für die HD und HDF-Postdilution ($\kappa = 0$) vereinfacht sich dieser Zusammenhang wieder zu:

$$K_d = Q_{bw} \frac{e^\gamma - 1}{e^\gamma - \frac{Q_{bw}}{Q_d}}, \gamma = k_0 A \frac{Q_d - Q_{bw}}{Q_{bw} Q_d}$$

Aus den Dialysat-Eingangskonzentrationen $c_{di}(1)$ und $c_{di}(2)$ und Dialysat-Ausgangskonzentration $c_{do}(1)$ und $c_{do}(2)$ kann die Clearance nach der folgenden Gleichung berechnet werden:
Die Rechen- und/oder Auswerteinheit 18A berechnet für den allgemeinen Fall der Hämodiafiltration die Clearance nach der Messung der Dialysat-Eingangskonzentrationen $c_{di}(1)$ und $c_{di}(2)$ und Dialysat-Ausgangskonzentration $c_{do}(1)$ und $c_{do}(2)$ anstelle nach Gleichung (4) wie folgt:

$$K_{m,tot} = (Q_d + Q_s + Q_f) \left( 1 - \frac{c_{do}(2) - c_{do}(1)}{c_{di}(2) - c_{di}(1)} \right) \qquad \text{Gleichung (4')}$$

wobei $Q_f$ die gesamte Filtrationsrate, d.h. die Summe aus Ultrafiltrationsrate $Q_{UF}$ und Substituatrate $Q_S$ ist.
Wenn die Dialysat-Eingangskonzentrationen $c_{di}(1)$ und $c_{di}(2)$ und Dialysat-Ausgangskonzentration $c_{do}(1)$ und $c_{do}(2)$ nicht stufenförmig (Stufenprofil), sondern kontinuierlich verändert werden (Pulsprofil) berechnet sich die Clearance wie folgt:

$$K_{m,tot} = (Q_d + Q_s + Q_f)\left(1 - \frac{\int_{t_2}^{t_3} \Delta c_{do}(t')dt'}{\int_{t_0}^{t1} \Delta c_{di}(t')dt'}\right)$$

wobei $\Delta cj$ die Höhe der LF-Variation über der Basislinie beschreibt.

Die oben genannten Gleichungen gelten für den Fall, dass im Dialysator Blut und Dialysat im Gegenstrom strömen. Wenn im Dialysator Blut und Dialysat hingegen im Gleichstrom strömen ergeben sich für den allgemeinen Fall der Hämodiafiltration die folgenden Zusammenhänge (Gotch, Replacement of Renal Function).

$$k_0 A = \frac{Q_b Q_d}{Q_d + Q_b} \ln\left(\frac{Q_b}{Q_b - K_d\left(1 + \frac{Q_b}{Q_d}\right)}\right)$$

$$k_0 A = -\frac{Q_b Q_d}{Q_d + Q_b} \ln\left(1 - K_d\left(\frac{1}{Q_b} + \frac{1}{Q_d}\right)\right)$$

$$K_d = Q_{bw} \frac{1 - e^{-\gamma}}{1 + \frac{Q_{bw}}{Q_d}}, \gamma = k_0 A \frac{Q_d + Q_{bw}}{Q_{bw} Q_d}$$

## Patentansprüche

1. Blutbehandlungsvorrichtung für eine extrakorporale Blutbehandlung mit
   einem Dialysator (1), der durch eine semipermeable Membran (2) in eine Blutkammer (3), die von Blut mit einem vorgegebenen Blutfluss $Q_b$ durchströmt werden kann, und einer Dialysatkammer (4), die von Dialysat mit einem vorgegebenen Dialysatfluss $Q_d$ durchströmt werden kann, unterteilt ist,
   einer zu der Blutkammer (3) führenden arteriellen Blutleitung (5) und einer von der Blutkammer (3) abgehenden venösen Blutleitung (7) und einer zu der Dialysatkammer (4) führenden Dialysatzuführleitung (9) und einer von der Dialysatkammer (4) abgehenden Dialysatabführleitung (10), und
   einer Vorrichtung (18) zur Ermittlung eines optimalen Dialysatflusses $Q_{dopt}$ für die extrakorporale Blutbehandlung, die eine Rechen- und/oder Auswerteinheit (18A) und eine Messeinrichtung (18B) zum Messen von mindestens einer für die Clearance K charakteristischen Größe aufweist, wobei die Rechen- und/oder Auswerteinheit (18A) derart konfiguriert ist, dass auf der Grundlage der mindestens einen für die Clearance charakteristischen Größe die Clearance ermittelt wird, und die Rechen- und/oder Auswerteinheit (18A) derart konfiguriert ist, dass der optimale Dialysatfluss $Q_{dopt}$ aus einem die Abhängigkeit der Clearance von der Dialysatrate beschreibenden Zusammenhang auf der Grundlage der gemessenen Clearance K oder der optimale Dialysatfluss $Q_{dopt}$ aus zumindest einer gemessenen Clearance K ermittelt wird,
   **dadurch gekennzeichnet, dass**
   die Blutbehandlungsvorrichtung eine Ventilanordnung (21) aufweist, die derart ausgebildet ist, dass zum Messen der Clearance K bei einer vorgegebenen Blutflussrate $Q_b$ und einer vorgegebenen Dialysatrate $Q_d$ eine Strömungsverbindung zwischen der arteriellen Blutleitung (5) und der venösen Blutleitung (7) herstellbar ist.

2. Blutbehandlungsvorrichtung nach Anspruch 1, **dadurch gekennzeichnet, dass** der die Abhängigkeit der Clearance K von dem Dialysatfluss $Q_d$ beschreibende Zusammenhang eine den Massentransferkoeffizienten $K_0A$ des Dialysators (1) der Blutbehandlungsvorrichtung berücksichtigende Funktion ist, wobei die Rechen- und/oder Auswerteinheit (18A) derart konfiguriert ist, dass aus der gemessenen Clearance K der Massentransferkoeffizient $K_0A$ des Dialysators (1) bestimmt wird und unter Berücksichtigung des Massentransferkoeffizienten $K_0A$ der optimale Dialysatflus $Q_{dopt}$ aus dem die Abhängigkeit der Clearance von dem Dialysatfluss beschreibenden Zusammenhang bestimmt wird.

3. Blutbehandlungsvorrichtung nach Anspruch 1 oder 2, **dadurch gekennzeichnet, dass** die Rechen- und/oder Auswerteinheit (18A) derart konfiguriert ist, dass der die Abhängigkeit der Clearance K von dem Dialysatfluss $Q_d$ beschreibende Zusammenhang auf der Grundlage der folgenden Gleichung bestimmt wird:

$$K = Q_b \frac{e^{K_0A\left(\frac{1}{Q_b}-\frac{1}{Q_d}\right)} - 1}{e^{K_0A\left(\frac{1}{Q_b}-\frac{1}{Q_d}\right)} - \frac{Q_b}{Q_d}}$$

,wobei $Q_d$ der Dialysatfluss, $Q_b$ der Blut(wasser)fluss und $K_0A$ der Massentransferkoeffizient des Dialysators ist.

4. Blutbehandlungsvorrichtung nach einem der Ansprüche 1 bis 3, **dadurch gekennzeichnet, dass** die Rechen- und/oder Auswerteinheit (18A) derart konfiguriert ist, dass der Massentransferkoeffizient $K_0A$ des Dialysators (1) nach der folgenden Gleichung berechnet wird,

$$K_OA = \frac{Q_b \cdot Q_d}{Q_b - Q_d} \ln\left(\frac{Q_d(Q_b - K)}{Q_b(Q_d - K)}\right)$$

,wobei $Q_d$ der Dialysatfluss, $Q_b$ der Blut(wasser)fluss und K die gemessene Clearance ist.

5. Blutbehandlungsvorrichtung nach einem der Ansprüche 1 bis 4, **dadurch gekennzeichnet, dass** die Rechen- und/oder Auswerteinheit (18A) derart konfiguriert ist, dass auf der Grundlage des die Abhängigkeit der Clearance K von dem Dialysatfluss $Q_d$ beschreibenden Zusammenhangs bei einem vorgegebenen Blutfluss $Q_b$ derjenige Dialysatfluss $Q_{dopt}$ ermittelt wird, bei dessen Erhöhung um einen bestimmten Wert die Erhöhung der Clearance einen bestimmten Wert nicht unterschreitet.

6. Blutbehandlungsvorrichtung nach einem der Ansprüche 1 bis 4, **dadurch gekennzeichnet, dass** die Rechen- und/oder Auswerteinheit (18A) derart konfiguriert ist, dass die Differenz zwischen dem Wert der bei einem vorgegebenen Dialysatfluss $Q_d$ gemessenen Clearance $K_m$ und einem vorgegebenen Wert für die Clearance berechnet wird, wobei ein Steuersignal zur Verringerung des Dialysatflusses $Q_d$ um einen vorgegebenen Betrag erzeugt wird, wenn die Differenz zwischen der gemessenen Clearance und der vorgegebenen Clearance positiv ist, und ein Steuersignal zur Erhöhung der Dialysatflusses $Q_d$ um einen vorgegebenen Betrag erzeugt wird, wenn die Differenz zwischen der gemessenen Clearance $K_m$ und der vorgegebenen Clearance negativ ist.

7. Blutbehandlungsvorrichtung nach Anspruch 6, **dadurch gekennzeichnet, dass** der vorgegebene Betrag, um den der Dialysatfluss $Q_d$ verringert oder erhöht wird, ein von dem Betrag der Differenz zwischen der gemessenen Clearance $K_m$ und der vorgegebenen Clearance abhängiger Betrag ist.

8. Blutbehandlungsvorrichtung nach Anspruch 7, **dadurch gekennzeichnet, dass** die Rechen- und/oder Auswerteinheit (18A) derart konfiguriert ist, dass die Berechnung der Differenz zwischen der gemessenen Clearance $K_m$ und der vorgegebenen Clearance und die Erzeugung eines Steuersignals zur Erhöhung oder Verringerung des Dialysatflusses $Q_d$ in mehreren aufeinanderfolgenden Schritten erfolgt.

9. Blutbehandlungsvorrichtung nach Anspruch 8, **dadurch gekennzeichnet, dass** die Berechnung der Differenz zwischen der gemessenen Clearance $K_m$ und der vorgegebenen Clearance und die Erzeugung eines Steuersignals zur Erhöhung oder Verringerung des Dialysatflusses in mehreren aufeinanderfolgenden Schritten solange erfolgt, bis der Betrag der Differenz zwischen der gemessenen Clearance $K_m$ und der vorgegebenen Clearance einen vorgegebenen Grenzwert unterschreitet.

10. Blutbehandlungsvorrichtung nach einem der Ansprüche 1 bis 4, **dadurch gekennzeichnet, dass** die Rechen- und/oder Auswerteinheit (18A) derart konfiguriert ist,
dass in einem ersten Schritt die Clearance $K_m$ bei einem vorgegebenen Dialysatfluss $Q_d$ gemessen wird;
dass ein Steuersignal zur Erhöhung des Dialysatflusses $Q_d$ um einen vorgegebenen Betrag erzeugt wird,
dass in einem zweiten Schritt die Clearance $K_m$ bei dem um den vorgegebenen Betrag erhöhten Dialysatfluss $Q_d$ gemessen wird,
dass die Veränderung der Clearance $K_m$ infolge der Veränderung des Dialysatflusses auf die Erfüllung eines Kriteriums für die Erhöhung oder Verringerung oder Beibehaltung des Dialysatflusses $Q_d$ überprüft wird, wobei
ein Steuersignal zur Erhöhung des Dialysatflusses $Q_d$ erzeugt wird, wenn das Kriterium zur Erhöhung des Dialysatflusses $Q_d$ erfüllt ist,
ein Steuersignal zur Verringerung des Dialysatflusses $Q_d$ erzeugt wird, wenn das Kriterium zur Verringerung des Dialysatflusses $Q_d$ erfüllt ist,
ein Steuersignal zur Beibehaltung des Dialysatflusses $Q_d$ erzeugt wird, wenn das Kriterium zur Beibehaltung des Dialysatflusses $Q_d$ erfüllt ist, und
dass bei der Erfüllung des Kriteriums für die Erhöhung oder Verringerung des Dialysatflusses $Q_d$ in aufeinanderfolgenden Schritten solange Steuersignale für die Erhöhung bzw. Verringerung des Dialysatflusses $Q_d$ erzeugt werden, bis das Kriterium für die Beibehaltung des Dialysatflusses $Q_d$ erfüllt ist.

11. Blutbehandlungsvorrichtung nach Anspruch 10, **dadurch gekennzeichnet, dass** die Rechen- und/oder Auswerteinheit (18A) derart konfiguriert ist, dass als Kriterium für die Erhöhung oder Verringerung oder Beibehaltung des Dialysatflusses $Q_d$ der Gradient der relativen Änderung der Clearance $K_m$ bei der relativen Änderung des Dialysatflusses $Q_d$ berechnet wird, wobei
ein Steuersignal zur Erhöhung des Dialysatflusses $Q_d$ erzeugt wird, wenn der Gradient der relativen Änderung der Clearance $K_m$ bei der relativen Änderung des Dialysatflusses $Q_d$ größer als ein erster Grenzwert ist,
ein Steuersignal zur Verringerung des Dialysatflusses $Q_d$ erzeugt wird, wenn der der Gradient der relativen Änderung der Clearance $K_m$ bei der relativen Änderung des Dialysatflusses $Q_d$ kleiner als ein zweiter Grenzwert ist, und
ein Steuersignal zur Beibehaltung des Dialysatflusses $Q_d$ erzeugt wird, wenn der der Gradient der relativen Änderung der Clearance $K_m$ bei der relativen Änderung des Dialysatflusses $Q_d$ kleiner als der erste Grenzwert und größer als der zweite Grenzwert ist.

12. Blutbehandlungsvorrichtung nach einem der Ansprüche 1 bis 11, **dadurch gekennzeichnet, dass** die Blutbehandlungsvorrichtung eine Steuereinheit (13) zur Einstellung des vorgegebenen Blutflusses $Q_b$ oder Dialysatflusses $Q_d$ aufweist, die das Steuersignal der Rechen- und/oder Auswerteinheit (18A) empfängt, so dass der optimale Dialysatfluss $Q_{dopt}$ eingestellt wird.

13. Verfahren zur Ermittlung eines optimalen Dialysatflusses $Q_{dopt}$ für eine extrakorporale Blutbehandlung mit einer extrakorporalen Blutbehandlungsvorrichtung, die einen Dialysator aufweist, der durch eine semipermeable Membran in eine Blutkammer, die von Blut mit einem vorgegebenen Blutfluss $Q_b$ durchströmt wird, und eine Dialysatkammer, die von Dialysat mit einem vorgegebenen Dialysatfluss $Q_d$ durchströmt wird, unterteilt ist, wobei der optimale Dialysatfluss $Q_{dopt}$ aus einem die Abhängigkeit der Clearance $K$ von der Dialysatsrate $Q_d$ beschreibenden Zusammenhang bestimmt wird, wobei
mindestens eine für die Clearance $K$ charakteristische Größe gemessen wird, wobei auf der Grundlage der mindestens einen für die Clearance charakteristischen Größe die Clearance $K$ ermittelt wird, und
der optimale Dialysatfluss $Q_{dopt}$ aus dem die Abhängigkeit der Clearance $K$ von dem Dialysatfluss $Q_d$ beschreibenden Zusammenhang auf der Grundlage der gemessenen Clearance $K$ oder der optimale Dialysatfluss $Q_{dopt}$ aus zumindest einer gemessenen Clearance $K$ ermittelt wird **dadurch gekennzeichnet, dass** das Verfahren nicht während der Blutbehandlung sondern während eines der Blutbehandlung vorausgehenden Spülvorgangs stattfindet, und dass zum Messen der Clearance $K$ eine Spülflüssigkeit über eine zu der Blutkammer (3) führende arterielle Blutleitung (5) in die Blutkammer (3) und über eine von der Blutkammer abgehende venösen Blutleitung (7) aus der Blutkammer gefördert wird, und dass eine Strömungsverbindung zwischen der arteriellen Blutleitung (5) und der venösen Blutleitung (7) hergestellt und die Spülflüssigkeit durch die Blutkammer rezirkuliert wird, wobei die Elektrolytkonzentration einer in die Dialysatkammer strömenden Spülflüssigkeit stromauf des Dialysators verändert und die Elektrolytkonzentration in der aus der Dialysatkammer strömenden Spülflüssigkeit gemessen wird.

14. Verfahren nach Anspruch 13, **dadurch gekennzeichnet, dass** der die Abhängigkeit der Clearance $K$ von dem Dialysatfluss $Q_d$ beschreibende Zusammenhang eine den Massentransferkoeffizienten $K_oA$ des Dialysators der Blutbehandlungsvorrichtung berücksichtigende Funktion ist, wobei aus der gemessenen Clearance der Massentransferkoeffizient $K_oA$ des Dialysators bestimmt wird und unter Berücksichtigung des Massentransferkoeffizienten

$K_0A$ der optimale Dialysatfluss $Q_{dopt}$ aus dem die Abhängigkeit der Clearance K von dem Dialysatfluss $Q_d$ beschreibenden Zusammenhang bestimmt wird.

15. Verfahren nach Anspruch 13 oder 14, **dadurch gekennzeichnet, dass** der die Abhängigkeit der Clearance K von dem Dialysatfluss $Q_d$ beschreibende Zusammenhang auf der Grundlage der folgenden Gleichung bestimmt wird:

$$K = Q_b \frac{e^{K_0A\left(\frac{1}{Q_b}-\frac{1}{Q_d}\right)} - 1}{e^{K_0A\left(\frac{1}{Q_b}-\frac{1}{Q_d}\right)} - \frac{Q_b}{Q_d}}$$

,wobei $Q_d$ der Dialysatfluss, $Q_b$ der Blut(wasser)fluss und $K_0A$ der Massentransferkoeffizient des Dialysators ist.

16. Verfahren nach einem der Ansprüche 13 bis 15, **dadurch gekennzeichnet, dass** der Massentransferkoeffizient $K_0A$ des Dialysators nach der folgenden Gleichung berechnet wird,

$$K_OA = \frac{Q_b \cdot Q_d}{Q_b - Q_d} \ln\left(\frac{Q_d(Q_b - K)}{Q_b(Q_d - K)}\right)$$

,wobei $Q_d$ der Dialysatfluss, $Q_b$ der Blut(wasser)fluss und K die gemessene Clearance ist.

17. Verfahren nach einem der Ansprüche 13 bis 16, **dadurch gekennzeichnet, dass** auf der Grundlage des die Abhängigkeit der Clearance K von dem Dialysatfluss $Q_d$ beschreibenden Zusammenhangs bei einer vorgegebenen Blutflussrate $Q_b$ derjenige Dialysatfluss $Q_{dopt}$ ermittelt wird, bei dessen Erhöhung um einen bestimmten Wert die Erhöhung der Clearance einen bestimmten Wert nicht unterschreitet.

18. Verfahren nach einem der Ansprüche 13 bis 16, **dadurch gekennzeichnet, dass** die Differenz zwischen dem Wert der bei einem vorgegebenen Dialysatfluss $Q_d$ gemessenen Clearance $K_m$ und einem vorgegebenen Wert für die Clearance berechnet wird, wobei der Dialysatfluss $Q_d$ um einen vorgegebenen Betrag verringert wird, wenn die Differenz zwischen der gemessenen Clearance $K_m$ und der vorgegebenen Clearance positiv ist, und der Dialysatfluss $Q_d$ um einen vorgegebenen Betrag erhöht wird, wenn die Differenz zwischen der gemessenen Clearance $K_m$ und der vorgegebenen Clearance negativ ist, wobei der vorgegebene Betrag, um den der Dialysatfluss verringert oder erhöht wird, ein von dem Betrag der Differenz zwischen der gemessenen Clearance $K_m$ und der vorgegebenen Clearance abhängiger Betrag ist, und die Berechnung der Differenz zwischen der gemessenen Clearance $K_m$ und der vorgegebenen Clearance und die Erzeugung eines Steuersignals zur Erhöhung oder Verringerung des Dialysatflusses $Q_d$ in mehreren aufeinanderfolgenden Schritten erfolgt.

19. Verfahren nach einem der Ansprüche 13 bis 16, **dadurch gekennzeichnet,**
    **dass** in einem ersten Schritt die Clearance $K_m$ bei einem vorgegebenen Dialysatfluss $Q_d$ gemessen wird;
    **dass** der Dialysatfluss $Q_d$ um einen vorgegebenen Betrag erhöht wird,
    **dass** in einem zweiten Schritt die Clearance $K_m$ bei dem um den vorgegebenen Betrag erhöhten Dialysatfluss $Q_d$ gemessen wird,
    **dass** die Veränderung der Clearance $K_m$ infolge der Veränderung des Dialysatflusses auf die Erfüllung eines Kriteriums für die Erhöhung oder Verringerung oder Beibehaltung des Dialysatflusses $Q_d$ überprüft wird, wobei der Dialysatfluss $Q_d$ erhöht wird, wenn das Kriterium zur Erhöhung des Dialysatflusses $Q_d$ erfüllt ist, der Dialysatfluss $Q_d$ verringert wird, wenn das Kriterium zur Verringerung des Dialysatflusses $Q_d$ erfüllt ist, der Dialysatfluss $Q_d$ beibehalten wird, wenn das Kriterium zur Beibehaltung des Dialysatflusses $Q_d$ erfüllt ist, und **dass** bei der Erfüllung des Kriteriums für die Erhöhung oder Verringerung des Dialysatflusses $Q_d$ in aufeinanderfolgenden Schritten solange der Dialysatfluss $Q_d$ erhöht oder veringert und die Veränderung der Clearance $K_m$ infolge der Veränderung des Dialysatflusses auf die Erfüllung des Kriteriums für die Erhöhung oder Verringerung oder Beibehaltung des Dialysatflusses $Q_d$ überprüft wird, bis das Kriterium für die Beibehaltung des Dialysatflusses $Q_d$ erfüllt ist.

**20.** Verfahren nach Anspruch 19, **dadurch gekennzeichnet, dass** als Kriterium für die Erhöhung oder Verringerung oder Beibehaltung des Dialysatflusses $Q_d$ der Gradient der relativen Änderung der Clearance $K_m$ bei der relativen Änderung des Dialysatflusses $Q_d$ berechnet wird, wobei

der Dialysatfluss $Q_d$ erhöht wird, wenn der Gradient der relativen Änderung der Clearance $K_m$ bei der relativen Änderung des Dialysatflusses $Q_d$ größer als ein erster Grenzwert ist,

der Dialysatfluss $Q_d$ verringert wird, wenn der Gradient der relativen Änderung der Clearance $K_m$ bei der relativen Änderung des Dialysatflusses $Q_d$ kleiner als ein zweiter Grenzwert ist,

der Dialysatfluss $Q_d$ beibehalten wird, wenn der Gradient der relativen Änderung der Clearance $K_m$ bei der relativen Änderung des Dialysatflusses $Q_d$ kleiner als der erste Grenzwert und größer als der zweite Grenzwert ist.

**Claims**

**1.** Blood treatment device for an extracorporeal blood treatment comprising

a dialyser (1) which is subdivided by a semipermeable membrane (2) into a blood chamber (3), which can be flowed through by blood at a predetermined blood flow $Q_b$, and a dialysate chamber (4), which can be flowed through by dialysate at a predetermined dialysate flow $Q_d$,

an arterial blood line (5) leading to the blood chamber (3) and a venous blood line (7) leaving the blood chamber (3) and a dialysate supply line (9) leading to the dialysate chamber (4) and a dialysate removal line (10) leaving the dialysate chamber (4),

a device (18) for determining an optimum dialysate flow $Q_{dopt}$ for the extracorporeal blood treatment, comprising a calculation and/or evaluation unit (18A) and a measurement device (18B) for measuring at least one value which is characteristic of the clearance K, the calculation and/or evaluation unit (18A) being configured in such a way that the clearance is determined on the basis of the at least one value which is characteristic of the clearance, and the calculation and/or evaluation unit (18A) being configured in such a way that the optimum dialysate flow $Q_{dopt}$ is determined from a relationship describing the dependence of the clearance on the dialysate rate on the basis of the measured clearance K or the optimum dialysate flow $Q_{dopt}$ is determined from at least one measured clearance K, **characterised in that**

the blood treatment device comprises a valve arrangement (21) which is formed in such a way that a flow connection between the arterial blood line (5) and the venous blood line (7) can be established for measuring the clearance K at a predetermined blood flow rate $Q_b$ and a predetermined dialysate rate $Q_d$.

**2.** Blood treatment device according to claim 1, **characterised in that** the relationship describing the dependence of the clearance K on the dialysate flow $Q_d$ is a function taking into account the mass transfer coefficient $K_oA$ of the dialyser (1) of the blood treatment device, the calculation and/or evaluation unit (18A) being configured in such a way that the mass transfer coefficient $K_oA$ of the dialyser (1) is determined from the measured clearance K and the optimum dialysate flow $Q_{dopt}$ is determined from the relationship describing the dependence of the clearance on the dialysate flow, taking into account the mass transfer coefficient $K_oA$.

**3.** Blood treatment device according to either claim 1 or claim 2, **characterised in that** the calculation and/or evaluation unit (18A) is configured in such a way that the relationship describing the dependence of the clearance K on the dialysate flow $Q_d$ is determined on the basis of the following equation:

$$K = Q_b \, \frac{e^{K_0 A \left( \frac{1}{Q_b} - \frac{1}{Q_d} \right)} - 1}{e^{K_0 A \left( \frac{1}{Q_b} - \frac{1}{Q_d} \right)} - \frac{Q_b}{Q_d}} \, ,$$

$Q_d$ being the dialysate flow, $Q_b$ being the blood (water) flow and $K_oA$ being the mass transfer coefficient of the dialyser.

**4.** Blood treatment device according to any of claims 1 to 3, **characterised in that** the calculation and/or evaluation unit (18A) is configured in such a way that the mass transfer coefficient $K_oA$ of the dialyser (1) is calculated using the following equation:

$$K_O A = \frac{Q_b \cdot Q_d}{Q_b - Q_d} \ln\left(\frac{Q_d(Q_b - K)}{Q_b(Q_d - K)}\right),$$

$Q_d$ being the dialysis flow, $Q_b$ being the blood (water) flow and K being the measured clearance.

5. Blood treatment device according to any of claims 1 to 4, **characterised in that** the calculation and/or evaluation unit (18A) is configured in such a way that, on the basis of the relationship describing the dependence of the clearance K on the dialysate flow $Q_d$ at a predetermined blood flow rate $Q_b$, the dialysate flow $Q_{dopt}$ is determined for which, when it is increased by a particular value, the increase in the clearance is not less than a particular value.

6. Blood treatment device according to any of claims 1 to 4, **characterised in that** the calculation and/or evaluation unit (18A) is configured in such a way that the difference between the value of the clearance $K_m$ measured at a predetermined dialysate flow $Q_d$ and a predetermined value for the clearance is calculated, a control signal for decreasing the dialysate flow $Q_d$ by a predetermined amount being generated if the difference between the measured clearance and the predetermined clearance is positive, and a control signal for increasing the dialysate flow $Q_d$ by a predetermined amount being generated if the difference between the measured clearance $K_m$ and the predetermined clearance is negative.

7. Blood treatment device according to claim 6, **characterised in that** the predetermined amount by which the dialysate flow $Q_d$ is decreased or increased is an amount dependent on the magnitude of the difference between the measured clearance $K_m$ and the predetermined clearance.

8. Blood treatment device according to claim 7, **characterised in that** the calculation and/or evaluation unit (18A) is configured in such a way that the calculation of the difference between the measured clearance $K_m$ and the predetermined clearance and the generation of a control signal for increasing or decreasing the dialysate flow $Q_d$ take place in a plurality of successive steps.

9. Blood treatment device according to claim 8, **characterised in that** the calculation of the difference between the measured clearance $K_m$ and the predetermined clearance and the generation of a control signal for increasing or decreasing the dialysate flow $Q_d$ take place in a plurality of successive steps until the magnitude of the difference between the measured clearance $K_m$ and the predetermined clearance undershoots a predetermined threshold.

10. Blood treatment device according to any of claims 1 to 4, **characterised in that** the calculation and/or evaluation unit (18A) is configured in such a way that
in a first step the clearance $K_m$ is measured at a predetermined dialysate flow $Q_d$;
a control signal for increasing the dialysate flow $Q_d$ by a predetermined amount is generated,
in a second step the clearance $K_m$ is measured at the dialysate flow $Q_d$ increased by the predetermined amount,
the change in the clearance $K_m$ as a result of the change in the dialysate flow is checked as to whether a criterion is met for increasing or decreasing or maintaining the dialysate flow $Q_d$,
a control signal for increasing the dialysate flow $Q_d$ being generated if the criterion for increasing the dialysate flow $Q_d$ is met,
a control signal for decreasing the dialysate flow $Q_d$ being generated if the criterion for decreasing the dialysate flow $Q_d$ is met,
a control signal for maintaining the dialysate flow $Q_d$ being generated if the criterion for maintaining the dialysate flow $Q_d$ is met, and
if the criterion for increasing or decreasing the dialysate flow $Q_d$ is met in successive steps, control signals for increasing or decreasing the dialysate flow $Q_d$ are generated until the criterion for maintaining the dialysate flow $Q_d$ is met.

11. Blood treatment device according to claim 10, **characterised in that** the calculation and/or evaluation unit (18A) is configured in such a way that the gradient of the relative change in the clearance $K_m$ for the relative change in the dialysate flow $Q_d$ is calculated as the criterion for increasing or decreasing or maintaining the dialysate flow $Q_d$,
a control signal for increasing the dialysate flow $Q_d$ being generated if the gradient of the relative change in the clearance $K_m$ for the relative change in the dialysate flow $Q_d$ is greater than a first threshold,
a control signal for decreasing the dialysate flow $Q_d$ being generated if the gradient of the relative change in the

clearance $K_m$ for the relative change in the dialysate flow $Q_d$ is less than a second threshold, and a control signal for maintaining the dialysate flow $Q_d$ being generated if the gradient of the relative change in the clearance $K_m$ for the relative change in the dialysate flow $Q_d$ is less than the first threshold and greater than the second threshold.

12. Blood treatment device according according to any of claims 1 to 11, **characterised in that** the blood treatment device comprises a control unit (13) for setting the predetermined blood flow $Q_b$ or dialysate flow $Q_d$, which receives the control signal of the calculation and/or evaluation unit (18A) so that the optimum dialysate flow $Q_{dopt}$ is set.

13. Method for determining an optimum dialysate flow $Q_{dopt}$ for an extracorporeal blood treatment using an extracorporeal blood treatment device comprising a dialyser which is subdivided by a semipermeable membrane into a blood chamber, which is flowed through by blood at a predetermined blood flow $Q_b$, and a dialysate chamber, which is flowed through by dialysate at a predetermined dialysate flow $Q_d$, the optimum dialysate flow $Q_{dopt}$ being determined from a relationship describing the dependence of the clearance K on the dialysate rate $Q_d$,
at least one value which is characteristic of the clearance K being measured, the clearance K being determined on the basis of the at least one value which is characteristic of the clearance, and
the optimum dialysate flow $Q_{dopt}$ being determined from the relationship describing the dependence of the clearance K on the dialysate flow $Q_d$ on the basis of the measured clearance $K_m$, or the optimum dialysate flow $Q_{dopt}$ is determined from at least one measured clearance K,
**characterized in that** the method is not being performed during the blood treatment but during a rinsing process in advance of the blood treatment, and that to measure the clearance K a rinsing liquid is conveyed into the blood chamber (3) via an arterial blood line (5) leading to the blood chamber (3) and out of the blood chamber via a venous blood line (7) leaving the blood chamber, and **in that** a flow connection is established between the arterial blood line (5) and the venous blood line (7), and the rinsing liquid is recirculated through the blood chamber, the electrolyte concentration of a rinsing liquid flowing into the dialysate chamber being changed upstream from the dialyser and the electrolyte concentration in the liquid flowing out of the dialysate chamber being measured.

14. Method according to claim 13, **characterised in that** the relationship describing the dependence of the clearance K on the dialysate flow $Q_d$ is a function taking into account the mass transfer coefficient $K_oA$ of the dialyser of the blood treatment device, the mass transfer coefficient $K_oA$ of the dialyser being determined from the measured clearance and the optimum dialysate flow $Q_{dopt}$ being determined from the relationship describing the dependence of the clearance K on the dialysate flow $Q_d$, taking into account the mass transfer coefficient $K_oA$.

15. Method according to either claim 13 or claim 14, **characterised in that** the relationship describing the dependence of the clearance K on the dialysate flow $Q_d$ is determined on the basis of the following equation:

$$K = Q_b \frac{e^{K_0 A\left(\frac{1}{Q_b} - \frac{1}{Q_d}\right)} - 1}{e^{K_0 A\left(\frac{1}{Q_b} - \frac{1}{Q_d}\right)} - \frac{Q_b}{Q_d}},$$

$Q_d$ being the dialysate flow, $Q_b$ being the blood (water) flow and $K_oA$ being the mass transfer coefficient of the dialyser.

16. Method according to any of claims 13 to 15, **characterised in that** the mass transfer coefficient $K_oA$ of the dialyser is calculated using the following equation:

$$K_O A = \frac{Q_b \cdot Q_d}{Q_b - Q_d} \ln\left(\frac{Q_d(Q_b - K)}{Q_b(Q_d - K)}\right),$$

$Q_d$ being the dialysis flow, $Q_b$ being the blood (water) flow and K being the measured clearance.

17. Method according to any of claims 13 to 16, **characterised in that**, on the basis of the relationship describing the dependence of the clearance K on the dialysate flow $Q_d$ at a predetermined blood flow rate $Q_b$, the dialysate flow $Q_{dopt}$ is determined for which, when it is increased by a particular value, the increase in the clearance is not less than a particular value.

18. Device according to any of claims 13 to 16, **characterised in that** the difference between the value of the clearance $K_m$ measured at a predetermined dialysate flow $Q_d$ and a predetermined value for the clearance is calculated, the dialysate flow $Q_d$ being decreased by a predetermined amount if the difference between the measured clearance $K_m$ and the predetermined clearance is positive, and the dialysate flow $Q_d$ being decreased by a predetermined amount if the difference between the measured clearance $K_m$ and the predetermined clearance is negative, the predetermined amount by which the dialysate flow is decreased or increased being an amount dependent on the magnitude of the difference between the measured clearance $K_m$ and the predetermined clearance, and the calculation of the difference between the measured clearance $K_m$ and the predetermined clearance and the generation of a control signal for increasing or decreasing the dialysate flow $Q_d$ taking place in a plurality of successive steps.

19. Method according to any of claims 13 to 16, **characterised in that**
in a first step the clearance $K_m$ is measured at a predetermined dialysate flow $Q_d$;
the dialysate flow $Q_d$ is increased by a predetermined amount,
in a second step the clearance $K_m$ is measured at the dialysate flow $Q_d$ increased by the predetermined amount,
the change in the clearance $K_m$ as a result of the change in the dialysate flow is checked as to whether a criterion is met for increasing or decreasing or maintaining the dialysate flow $Q_d$,
the dialysate flow $Q_d$ being increased if the criterion for increasing the dialysate flow $Q_d$ is met,
the dialysate flow $Q_d$ being decreased if the criterion for decreasing the dialysate flow $Q_d$ is met,
the dialysate flow $Q_d$ being maintained if the criterion for maintaining the dialysate flow $Q_d$ is met, and
if the criterion for increasing or decreasing the dialysate flow $Q_d$ is met in successive steps, the dialysate flow $Q_d$ is increased or decreased and the change in the clearance $K_m$ as a result of the change in the dialysate flow is checked as to whether the criterion is met for increasing or decreasing or maintaining the dialysate flow $Q_d$ until the criterion for maintaining the dialysate flow $Q_d$ is met.

20. Method according to claim 19, **characterised in that** the gradient of the relative change in the clearance $K_m$ for the relative change in the dialysate flow $Q_d$ is calculated as the criterion for increasing or decreasing or maintaining the dialysate flow $Q_d$,
the dialysate flow $Q_d$ being increased if the gradient of the relative change in the clearance $K_m$ for the relative change in the dialysate flow $Q_d$ is greater than a first threshold,
the dialysate flow $Q_d$ being decreased if the gradient of the relative change in the clearance $K_m$ for the relative change in the dialysate flow $Q_d$ is less than a second threshold, and
the dialysate flow $Q_d$ being maintained if the gradient of the relative change in the clearance $K_m$ for the relative change in the dialysate flow $Q_d$ is less than the first threshold and greater than the second threshold.

**Revendications**

1. Dispositif de traitement de sang pour un traitement de sang extracorporel avec un dialyseur (1), qui est divisé par une membrane semi-perméable (2) en une chambre à sang (3), qui peut être traversée par du sang avec un flux de sang $Q_b$ spécifié, et une chambre à dialysat (4), qui peut être traversée par du dialysat avec un flux de dialysat $Q_d$ spécifié,
un conduit de sang artériel (5) menant à la chambre à sang (3) et un conduit de sang veineux (7) sortant de la chambre à sang (3) et un conduit d'amenée de dialysat (9) menant vers la chambre à dialysat (4) et un conduit d'évacuation de dialysat (10) sortant de la chambre à dialysat (4), et
un dispositif (18) servant à déterminer un flux de dialysat optimal $Q_{dopt}$ pour le traitement de sang extracorporel, qui présente une unité de calcul et/ou d'analyse (18A) et un système de mesure (18B) servant à mesurer au moins une grandeur caractéristique de la clairance K, dans lequel l'unité de calcul et/ou d'analyse (18A) est configurée de telle manière que la clairance est déterminée sur la base de l'au moins une grandeur caractéristique de la clairance et l'unité de calcul et/ou d'analyse (18A) est configurée de telle manière que le flux de dialysat optimal $Q_{dopt}$ est déterminé à partir d'un rapport décrivant la dépendance de la clairance et du débit de dialysat sur la base de la clairance K mesurée ou le flux de dialysat optimal $Q_{dopt}$ est déterminé à partir d'au moins une clairance K mesurée,
**caractérisé en ce que**
le dispositif de traitement de sang présente un ensemble de soupapes (21), qui est réalisé de telle manière que pour mesurer la clairance K pour un débit de flux de sang $Q_b$ spécifié et un débit de dialysat $Q_d$ spécifié, une communication fluidique peut être établie entre le conduit de sang artériel (5) et le conduit de sang veineux (7).

2. Dispositif de traitement de sang selon la revendication 1, **caractérisé en ce que** le rapport décrivant la dépendance de la clairance K du flux de dialysat $Q_d$ est une fonction tenant compte du coefficient de transfert de masse $K_oA$ du dialyseur (1) du dispositif de traitement de sang, dans lequel l'unité de calcul et/ou d'analyse (18A) est configurée de telle manière que le coefficient de transfert de masse $K_oA$ du dialyseur (1) est déterminé à partir de la clairance K mesurée et le flux de dialysat optimal $Q_{dopt}$ est déterminé à partir du rapport décrivant la dépendance de la clairance et du flux de dialysat en tenant compte du coefficient de transfert de masse $K_oA$.

3. Dispositif de traitement de sang selon la revendication 1 ou 2, **caractérisé en ce que** l'unité de calcul et/ou d'analyse (18A) est configurée de telle manière que le rapport décrivant la dépendance de la clairance K du flux de dialysat $Q_d$ est déterminé sur la base de l'équation suivante :

$$K = Q_b \frac{e^{K_0A\left(\frac{1}{Q_b}-\frac{1}{Q_d}\right)} - 1}{e^{K_0A\left(\frac{1}{Q_b}-\frac{1}{Q_d}\right)} - \frac{Q_b}{Q_d}},$$

dans lequel $Q_d$ est le flux de dialysat, $Q_b$ est le flux de (sérum) de sang et $K_oA$ est le coefficient de transfert de masse du dialyseur.

4. Dispositif de traitement de sang selon l'une quelconque des revendications 1 à 3, **caractérisé en ce que** l'unité de calcul et/ou d'analyse (18A) est configurée de telle manière que le coefficient de transfert de masse $K_oA$ du dialyseur (1) est calculé selon l'équation suivante :

$$K_OA = \frac{Q_b \cdot Q_d}{Q_b - Q_d} \ln\left(\frac{Q_d(Q_b - K)}{Q_b(Q_d - K)}\right),$$

dans lequel $Q_d$ est le flux de dialysat, $Q_b$ est le flux (de sérum) de sang et K est la clairance mesurée.

5. Dispositif de traitement de sang selon l'une quelconque des revendications 1 à 4, **caractérisé en ce que** l'unité de calcul et/ou d'analyse (18A) est configurée de telle manière qu'est déterminé, sur la base du rapport décrivant la dépendance de la clairance K du flux de dialysat $Q_d$, pour un flux de sang $Q_b$ spécifié, le flux de dialysat $Q_{dopt}$, lors de l'augmentation duquel d'une valeur définie, l'augmentation de la clairance n'est pas inférieure à une valeur définie.

6. Dispositif de traitement de sang selon l'une quelconque des revendications 1 à 4, **caractérisé en ce que** l'unité de calcul et/ou d'analyse (18A) est configurée de telle manière que la différence entre la valeur de la clairance mesurée $K_m$ pour un flux de dialysat $Q_d$ spécifié et une valeur spécifiée pour la clairance est calculée, dans lequel un signal de commande est produit pour diminuer le flux de dialysat $Q_d$ d'une valeur spécifiée quand la différence entre la clairance mesurée et la clairance spécifiée est positive, et un signal de commande est produit pour augmenter le flux de dialysat $Q_d$ d'une valeur spécifiée quand la différence entre la clairance mesurée $K_m$ et la clairance spécifiée est négative.

7. Dispositif de traitement de sang selon la revendication 6, **caractérisé en ce que** la valeur spécifiée, de laquelle le flux de dialysat $Q_d$ est diminué ou augmenté, est une valeur dépendant de la valeur de la différence entre la clairance mesurée $K_m$ et la clairance spécifiée.

8. Dispositif de traitement de sang selon la revendication 7, **caractérisé en ce que** l'unité de calcul et/ou d'analyse (18A) est configurée de telle manière que le calcul de la différence entre la clairance mesurée $K_m$ et la clairance spécifiée et la production d'un signal de commande pour augmenter ou diminuer le flux de dialysat $Q_d$ sont effectués lors de plusieurs étapes se suivant les unes les autres.

9. Dispositif de traitement de sang selon la revendication 8, **caractérisé en ce que** le calcul de la différence entre la clairance mesurée $K_m$ et la clairance spécifiée et la production d'un signal de commande servant à augmenter ou à diminuer le flux de dialysat sont effectués lors de plusieurs étapes se suivant les unes les autres jusqu'à ce que la valeur de la différence entre la clairance mesurée $K_m$ et la clairance spécifiée soit inférieure à une valeur limite spécifiée.

10. Dispositif de traitement de sang selon l'une quelconque des revendications 1 à 4, **caractérisé en ce que** l'unité de calcul et/ou d'analyse (18A) est configurée de telle manière
que lors d'une première étape, la clairance $K_m$ est mesurée pour un flux de dialysat $Q_d$ spécifié ;
qu'un signal de commande est produit pour augmenter le flux de dialysat $Q_d$ d'une valeur spécifiée,
que lors d'une deuxième étape, la clairance $K_m$ est mesurée pour le flux de dialysat $Q_d$ augmenté de la valeur spécifiée,
que la modification de la clairance $K_m$ suite à la modification du flux de dialysat est vérifiée quant au respect d'un critère de l'augmentation ou de la diminution ou du maintien du flux de dialysat $Q_d$, dans lequel
un signal de commande est produit pour augmenter le flux de dialysat $Q_d$ quand le critère concernant l'augmentation du flux de dialysat $Q_d$ est rempli,
un signal de commande est produit pour diminuer le flux de dialysat $Q_d$ quand le critère concernant la diminution du flux de dialysat $Q_d$ est rempli,
un signal de commande est produit pour le maintien du flux de dialysat $Q_d$ quand le critère concernant le maintien du flux de dialysat $Q_d$ est rempli, et
que lors du respect du critère de l'augmentation ou de la diminution du flux de dialysat $Q_d$, des signaux de commande pour l'augmentation ou la diminution du flux de dialysat $Q_d$ sont produits lors d'étapes se suivant les unes les autres jusqu'à ce que le critère du maintien du flux de dialysat $Q_d$ soit rempli.

11. Dispositif de traitement de sang selon la revendication 10, **caractérisé en ce que** l'unité de calcul et/ou d'analyse (18A) est configurée de telle manière que le gradient du changement relatif de la clairance $K_m$ lors du changement relatif du flux de dialysat $Q_d$ est calculé en tant que critère de l'augmentation ou de la diminution ou du maintien du flux de dialysat $Q_d$, dans lequel
un signal de commande est produit pour augmenter le flux de dialysat $Q_d$ quand le gradient du changement relatif de la clairance $K_m$ pour le changement relatif du flux de dialysat $Q_d$ est supérieur à une première valeur limite,
un signal de commande est produit pour diminuer le flux de dialysat $Q_d$ quand le gradient du changement relatif de la clairance $K_m$ lors du changement relatif du flux de dialysat $Q_d$ est inférieur à une deuxième valeur limite, et
un signal de commande est produit pour maintenir le flux de dialysat $Q_d$ quand le gradient du changement relatif de la clairance $K_m$ lors du changement relatif du flux de dialysat $Q_d$ est inférieur à la première valeur limite ou est supérieur à la deuxième valeur limite.

12. Dispositif de traitement de sang selon l'une quelconque des revendications 1 à 11, **caractérisé en ce que** le dispositif de traitement de sang présente une unité de commande (13) servant à régler le flux de sang $Q_b$ spécifié ou le flux de dialysat $Q_d$, qui reçoit le signal de commande de l'unité de calcul et/ou d'analyse (18A) de sorte que le flux de dialysat optimal $Q_{dopt}$ est réglé.

13. Procédé servant à déterminer un flux de dialysat optimal $Q_{dopt}$ pour un traitement de sang extracorporel avec un dispositif de traitement de sang extracorporel, qui présente un dialyseur, qui est divisé par une membrane semi-perméable en une chambre à sang, qui est traversée par du sang avec un flux de sang $Q_b$ spécifié, et une chambre à dialysat, qui est traversé par du dialysat avec un flux de dialysat $Q_d$ spécifié, dans lequel le flux de dialysat optimal $Q_{dopt}$ est déterminé à partir d'un rapport décrivant la dépendance de la clairance K du débit de dialysat $Q_d$,

dans lequel

au moins une grandeur caractéristique de la clairance K est mesurée, dans lequel la clairance K est déterminée sur la base de l'au moins une grandeur caractéristique de la clairance, et

le flux de dialysat optimal $Q_{dopt}$ est déterminé à partir du rapport décrivant la dépendance de la clairance K du flux de dialysat $Q_d$ sur la base de la clairance K mesurée ou le flux de dialysat optimal $Q_{dopt}$ est déterminé à partir d'au moins une clairance mesurée K,

**caractérisé en ce que** le procédé n'a pas lieu pendant le traitement du sang mais pendant une opération de rinçage précédant le traitement du sang, et que pour mesurer la clairance K, un liquide de rinçage est refoulé dans la chambre à sang (3) par l'intermédiaire d'un conduit de sang artériel (5) menant vers la chambre à sang (3) et hors de la chambre à sang par l'intermédiaire d'un conduit de sang veineux (7) sortant de la chambre à sang, et qu'une communication fluidique est établie entre le conduit de sang artériel (5) et le conduit de sang veineux (7) et le liquide de rinçage est remis en circulation à travers la chambre à sang, dans lequel la concentration d'électrolyte d'un liquide de rinçage s'écoulant dans la chambre à dialysat est modifiée en amont du dialyseur et la concentration d'électrolyte dans le liquide de rinçage s'écoulant hors de la chambre à dialysat est mesurée.

14. Procédé selon la revendication 13, **caractérisé en ce que** le rapport décrivant la dépendance de la clairance K du flux de dialysat $Q_d$ est une fonction tenant compte du coefficient de transfert de masse $K_oA$ du dialyseur du dispositif de traitement de sang, dans lequel le coefficient de transfert de masse $K_oA$ du dialyseur est déterminé à partir de la clairance mesurée et le flux de dialysat optimal $Q_{dopt}$ est déterminé à partir du rapport décrivant la dépendance de la clairance K du flux de dialysat $Q_d$ en tenant compte du coefficient de transfert de masse $K_oA$.

15. Procédé selon la revendication 13 ou 14, **caractérisé en ce que** le rapport décrivant la dépendance de la clairance K et du flux de dialysat $Q_d$ est déterminé sur la base de l'équation suivante :

$$K = Q_b \frac{e^{K_0 A\left(\frac{1}{Q_b}-\frac{1}{Q_d}\right)} - 1}{e^{K_0 A\left(\frac{1}{Q_b}-\frac{1}{Q_d}\right)} - \frac{Q_b}{Q_d}} \quad ,$$

dans lequel $Q_d$ est le flux de dialysat, $Q_b$ est le flux de (sérum) de sang et $K_oA$ est le coefficient de transfert de masse du dialyseur.

16. Procédé selon l'une quelconque des revendications 13 à 15, **caractérisé en ce que** le coefficient de transfert de masse $K_oA$ du dialyseur est calculé selon l'équation suivante :

$$K_O A = \frac{Q_b \cdot Q_d}{Q_b - Q_d} \ln\left(\frac{Q_d(Q_b - K)}{Q_b(Q_d - K)}\right) \quad ,$$

dans lequel $Q_d$ est le flux de dialysat, $Q_b$ est le flux de (sérum) de sang et K est la clairance mesurée.

17. Procédé selon l'une quelconque des revendications 13 à 16, **caractérisé en ce qu'**est déterminé sur la base du rapport décrivant la dépendance de la clairance K du flux de dialysat $Q_d$, pour un débit de flux de sang $Q_b$ spécifié, le flux de dialysat $Q_{dopt}$, lors de l'augmentation duquel d'une valeur définie, l'augmentation de la clairance n'est pas inférieure à une valeur définie.

**18.** Procédé selon l'une quelconque des revendications 13 à 16, **caractérisé en ce que** la différence entre la valeur de la clairance mesurée $K_m$ pour un flux de dialysat $Q_d$ spécifié et une valeur spécifiée pour la clairance est calculée, dans lequel le flux de dialysat $Q_d$ est diminué d'une valeur spécifiée quand la différence entre la clairance mesurée $K_m$ et la clairance spécifiée est positive, et le flux de dialysat $Q_d$ est augmenté d'une valeur spécifiée quand la différence entre la clairance mesurée $K_m$ et la clairance spécifiée est négative, dans lequel la valeur spécifiée, de laquelle le flux de dialysat est diminué ou augmenté, est une valeur dépendant de la valeur de la différence entre la clairance mesurée $K_m$ et la clairance spécifiée et le calcul de la différence entre la clairance mesurée $K_m$ et la clairance spécifiée et la production d'un signal de commande pour augmenter ou diminuer le flux de dialysat $Q_d$ sont effectués lors de plusieurs étapes se suivant les unes les autres.

**19.** Procédé selon l'une quelconque des revendications 13 à 16, **caractérisé en ce que** lors d'une première étape, la clairance $K_m$ est mesurée pour un flux de dialysat $Q_d$ spécifié ;
que le flux de dialysat $Q_d$ est augmenté d'une valeur spécifiée,
que lors d'une deuxième étape, la clairance $K_m$ est mesurée pour le flux de dialysat $Q_d$ augmenté de la valeur spécifiée,
que la modification de la clairance $K_m$ suite à la modification du flux de dialysat est vérifiée quant au respect d'un critère de l'augmentation ou de la diminution ou du maintien du flux de dialysat $Q_d$, dans lequel
le flux de dialysat $Q_d$ est augmenté quand le critère concernant l'augmentation du flux de dialysat $Q_d$ est respecté,
le flux de dialysat $Q_d$ est diminué quand le critère concernant la diminution du flux de dialysat $Q_d$ est respecté,
le flux de dialysat $Q_d$ est maintenu quand le critère concernant le maintien du flux de dialysat $Q_d$ est respecté, et
que lors du respect du critère de l'augmentation ou de la diminution du flux de dialysat $Q_d$, le flux de dialysat $Q_d$ est augmenté ou diminué lors d'étapes se suivant les unes les autres et la modification de la clairance $K_m$ suite à la modification du flux de dialysat est vérifiée quant au respect du critère de l'augmentation ou de la diminution ou du maintien du flux de dialysat $Q_d$ jusqu'à ce que le critère du maintien du flux de dialysat $Q_d$ soit respecté.

**20.** Procédé selon la revendication 19, **caractérisé en ce que** le gradient du changement relatif de la clairance $K_m$ lors du changement relatif du flux de dialysat $Q_d$ est calculé en tant que critère de l'augmentation ou de la diminution ou du maintien du flux de dialysat $Q_d$, dans lequel
le flux de dialysat $Q_d$ est augmenté quand le gradient du changement relatif de la clairance $K_m$ lors du changement relatif du flux de dialysat $Q_d$ est supérieur à une première valeur limite,
le flux de dialysat $Q_d$ est diminué quand le gradient du changement relatif de la clairance $K_m$ lors du changement relatif du flux de dialysat $Q_d$ est inférieur à une deuxième valeur limite,
le flux de dialysat $Q_d$ est maintenu quand le gradient du changement relatif de la clairance $K_m$ lors du changement relatif du flux de dialysat $Q_d$ est inférieur à la première valeur limite et est supérieur à la deuxième valeur limite.

# Fig. 1

Fig. 2

$$Q_{d,1} = Q_{b,}$$

Messung $K_1 (Q_{d,1})$

$$Q_{d,2} := Q_{d,1} + \Delta Q_d$$

neuen Fluss $Q_{d,2}$ einstellen

Messung $K_2 (Q_{d,2})$

$c2 < \delta(K_1, K_2) < c1$     $\delta(K_1, K_2) > c1$

$$Q_{d,1} := Q_{d,2} \; ; \; K_1 := K_2$$
$$Q_{d,2} := Q_{d,2} + \Delta Q_d$$

$\delta(K_1, K_2) < c2$

$$Q_{d,1} := Q_{d,2} \; ; \; K_1 := K_2$$
$$Q_{d,2} := Q_{d,2} - \Delta Q_d$$

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3938662 A1
- US 5100554 A
- DE 19747360 A1
- US 6156002 A
- US 5092836 A
- DE 102006045437 A1
- US 2010042035 A1
- WO 2007140993 A1